(19) Europäisches Patentamt
European Patent Office
Office européen des brevets

(11) Numéro de publication: **0 275 729 B1**

# (12) FASCICULE DE BREVET EUROPEEN

(45) Date de publication de fascicule du brevet: **08.05.91**

(51) Int. Cl.[5]: **C07J 9/00**, C07J 7/00, C07J 41/00

(21) Numéro de dépôt: **87402740.2**

(22) Date de dépôt: **03.12.87**

(54) **Nouveaux produits stéroides comportant, en position 23, un radical cétonique, leur procédé de préparation, leur application à la préparation de produits de la série des 20-cétoprégnanes et des intermédiaires de cette application.**

(30) Priorité: **05.12.86 FR 8617051**

(43) Date de publication de la demande:
**27.07.88 Bulletin 88/30**

(45) Mention de la délivrance du brevet:
**08.05.91 Bulletin 91/19**

(84) Etats contractants désignés:
**AT BE CH DE ES FR GB GR IT LI LU NL SE**

(56) Documents cités:
**US-A- 2 180 095**
**US-A- 2 445 006**
**US-A- 2 705 232**
**US-A- 4 500 460**

**JOURNAL OF THE AMERICAN CHEMICAL SOCIETY, vol. 74, no. 23, 5 décembre 1952, pages 5814-5818, Washington, D.C., US; R.B. TURNER et al.: "Steroids derived from bile acids. XIX. Barbier-Wieland degradation in the 11-keto series"**

(73) Titulaire: **ROUSSEL-UCLAF**
**35, boulevard des Invalides**
**F-75007 Paris(FR)**

(72) Inventeur: **Vivat, Michel**
**14, Chemin de l'Autostrade**
**F-77400 Lagny-Sur-Marne(FR)**
Inventeur: **Buendia, Jean**
**3bis, Impasse Emilie**
**F-94170 Le Perreux-Sur-Marne(FR)**

(74) Mandataire: **Bourgouin, André et al**
**Département des Brevets ROUSSEL UCLAF**
**111, route de Noisy B.P. no 9**
**F-93230 Romainville(FR)**

**EP 0 275 729 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux produits stéroïdes comportant en position 23 un radical cétonique, leur procédé de préparation, leur application à la préparation de produits de la série des 20-céto pregnane et des intermédiaires de cette application.

Dans le brevet américain US-A-2.705.232 sont décrits des produits dérivés de l'acide alpha-oxo cholanique. Cependant ces produits ne comportent pas de fonctions réactives en position 11 ou 12.

La présente invention a pour objet les produits de formule générale (I) :

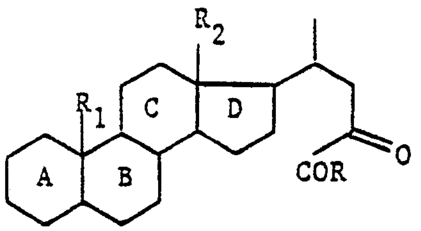

(I)

dans laquelle $R_1$ représente un atome d'hydrogène ou un radical méthyle, $R_2$ représente un radical méthyle ou éthyle, les noyaux A, B, C, D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkoxy renfermant de 1 à 4 atomes de carbone R représente un atome d'halogène, un radical hydroxyle, un radical alkoxy ayant de 1 à 6 atomes de carbone, un radical aralkoxy ayant de 7 à 15 atomes de carbone, un radical :

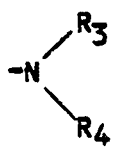

dans lequel $R_3$ et $R_4$, identiques ou différents représentent un atome d'hydrogène, un radical alkyle ayant de 1 à 6 atomes de carbone, un radical aralkyle ayant de 7 à 15 atomes de carbone ou $R_3$ et $R_4$ forment ensemble avec l'atome d'azote auquel ils sont liés un hétérocycle comportant éventuellement un autre hétéroatome choisi parmi les atomes d'azote et d'oxygène, ou R représente un radical alkylthio ayant de 1 à 6 atomes de carbone, un radical arylthio ou aralkylthio ayant au plus 15 atomes de carbone à l'exception du produit dans lequel $R_1$ et $R_2$ représentent chacun un radical méthyl, R représente un radical méthoxy, le noyau A porte une position 3-béta acétoxy et le noyau B porte une double liaison 5(6) et étant entendu que le noyau C comporte soit une fonction cétonique éventuellement protégée en position 11 soit une fonction hydroxyle éventuellement protégée en position 12.

Lorsque les cycles A, B, C et D portent une ou plusieurs doubles liaisons il s'agit de préférence de doubles liaisons en 1(2), 4(5), 5(6) ou 9(11) d'un système de doubles liaisons coniuguées en 3(4) et 5(6) ou en 4(5) et 6(7) ou d'un système aromatique de trois doubles liaisons 1, 3, 5 ou d'un système de trois doubles liaisons 1(2), 4(5), 6(7). On utilise cependant de préférence des produits de comportant pas de double liaison.

Lorsque les cycles A, B, C et D sont substitués par une ou plusieurs fonctions hydroxyles, il s'agit de préférence d'une ou plusieurs fonctions hydroxyles en 3, en 6, en 7 et/ou 12.

Lorsque les cycles A, B, C et D sont substitués par une ou plusieurs fonctions cétones, il s'agit de - préférence d'une fonction cétone en 3, en 7 et/ou en 11.

Lorsque les cycles A, B, C et D sont substitués par un ou plusieurs atomes d'halogènes, il s'agit de préférence d'un atome de fluor, de chlore ou de brome, en position 6 ou 9 alpha par exemple.

Lorsque les cycles A, B, C et D sont substitués par un ou plusieurs radicaux alkyles, il s'agit de préférence du radical méthyle ou éthyle en 2, en 6, en 7, en 16 alpha ou en 16 béta.

Lorsque les cycles A, B, C et D sont substitués par un ou plusieurs radicaux alkyloxy il s'agit de préférence d'un radical méthoxy ou éthoxy en 3 ou 11 béta.

Les groupements hydroxyles peuvent être protégés selon les modes habituels connus dans la

littérature. On peut par exemple citer les groupements acétonides, les carbonates cycliques, les orthoesters,, les sulfites cycliques, l'éther formé avec le tétrahydropyrannyle, le groupement trityle, benzyle, les radicaux acyles, tels qu'acétyle, succinyle ou formyle.

Les groupements cétoniques peuvent de même être protégés par les groupements protecteurs classiques, tels que les cétals, plus spécialement l'éthylène cétal, les thioacétals, les hémithioacétals, les éthers d'énols, les acétates d'énols, les énamines, les oximes.

On préfère cependant les groupements cétals et spécialement l'éthylène cétal pour protéger les groupements cétoniques. Lorsque les produits de formule 1 comportent un groupement cétonique en position 3, ce groupement est très préférentiellement protégé.

R peut représenter un atome d'halogène, de préférence un atome de chlore ou de brome, R peut également représenter un radical alkoxy tel que méthoxy ou éthoxy de préférence mais également propyloxy, isopropyloxy, butyloxy, sec-butyloxy, tert-butyloxy, pentyloxy, hexyloxy, R peut également représenter un radical benzyloxy, phényléthyloxy.

Les radicaux $R_3$ et $R_4$ identiques ou différents peuvent représenter un atome d'hydrogène ou des radicaux méthyle, éthyle, propyle, isopropyle, butyle, sec-butyle, tert-butyle, pentyle, hexyle, benzyle, soit $R_3$ et $R_4$ forment ensemble avec l'atome d'azote auquel ils sont liés un radical morpholine, pipéridine, pyrrolidine. R peut également représenter un radical méthylthio ou éthylthio ou un radical alkylthio dérivé des radicaux alkyle ou alkoxy indiqués ci-dessus. R peut également représenter un radical phénylthio ou benzylthio.

L'invention a plus particulièrement pour objet les produits de formule générale 1 telle que définie ci-dessus dans laquelle $R_1$ et $R_2$ représentent chacun un radical méthyle et les noyaux A, B, C, D portent en position 3 une fonction hydroxyle éventuellement protégée et, éventuellement, une ou plusieurs autres fonctions choisies parmi les fonctions hydroxyles éventuellement protégées en position 6,7 et 12 et les fonctions cétones éventuellement protégées en position 7 et 11 et R représente un radical hydroxy, un radical alkoxy ayant au plus 4 atomes de carbone ou un radical:

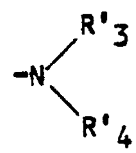

dans lequel $R'_3$ et $R'_4$ représentent un atome d'hydrogène, un radical alkyle ayant au plus 4 atomes de carbone ou $R'_3$ et $R'_4$ forment ensemble avec l'atome d'azote auquel ils sont liés un radical pipéridino, pyrrolidino ou morpholino et plus spécialement les produits de formule générale 1 telle que définie ci-dessus dans laquelle les noyaux A, B, C, D portent en position 3 un radical hydroxyle éventuellement protégé et R représente un radical hydroxyle, méthoxy ou éthoxy ou un radical morpholino.

Parmi les produits préférés une sous-famille de produits particulièrement avantageux est constituée par les produits de formule générale 1 telle que définie ci-dessus, dans laquelle les noyaux A, B, C, D portent en position 3 un radical hydroxyle éventuellement protégé et, éventuellement, une ou plusieurs autres fonctions choisies parmi les fonctions hydroxyles éventuellement protégées en position 6, 7 et/ou 12 et les fonctions cétones éventuellement protégées, en position 11 ou 7 et 11.

Dans cette dernière famille, on peut citer les produits comportant, comme squelette les cycles A, B, C, D, les produits dérivés des acides biliaires naturels ou hémi-synthétiques. Ces produits peuvent être énumérés par le tableau suivant:

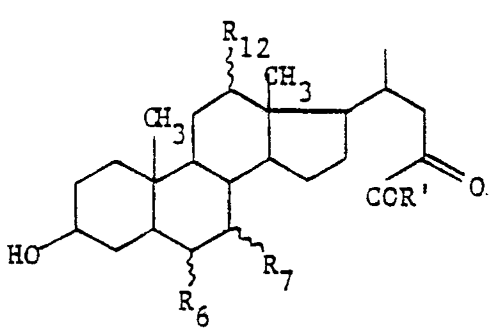

dans laquelle R' représente un radical hydroxyle, méthoxy, éthoxy ou morpholino et $R_6$, $R_7$ et $R_{12}$ ont les significations suivantes:

| $R_6$ | $R_7$ | $R_{12}$ |
|---|---|---|
| H | OH alpha | OH alpha |
| H | OH béta | OH alpha |
| H | H | OH alpha |
| H | H | OH alpha |
| H | OH alpha | OH alpha |

Dans ces produits, le ou les radicaux hydroxyles peuvent également être protégés notamment le radical hydroxyle en position 3. Le groupement protecteur préféré peut être le groupement acétyle ou le groupement formyle.

Parmi les produits comportant une ou plusieurs fonctions cétones, on préfére les produits suivants:

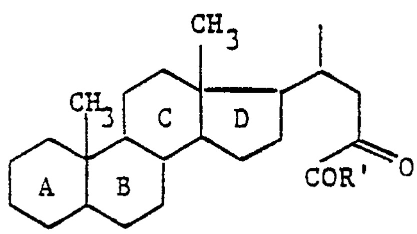

dans lesquels R' représente un radical hydroxyle, méthoxy, éthoxy ou morpholino, et les substituants en position 3, 7, 11 et 12 ont les significations suivantes:

- 3 OH alpha, 7 céto, 12 OH alpha,
- 3 OH alpha, 11 céto
- 3 OH alpha, 7 OH alpha, 12 céto
- 3 OH, 11 céto.

Bien entendu, comme précédemment, les radicaux hydroxyles peuvent être protégés. il en est de même des radicaux cétoniques en position 7 ou 12. Le groupement protecteur préféré du groupement cétonique est un cétal cyclique ou non cyclique.

Les produits préférés sont les produits décrits ci-après dans les exemples, et notamment:

- La (3 alpha, 5 béta) 4-[3-(acétyloxy) 11, 23, 24-trioxo-cholan 24-yi] morpholine.
- L'acide (3 alpha, 5 béta) 11, 23-dioxo 3-hydroxy cholan 24-oïque.
- L'ester méthylique de l'acide 3 alpha-hydroxy 11, 23-dioxo 5-béta-cholan 24-oïque.
- L'acide (3 alpha, 5 béta) 3-(acétyloxy) 11, 23-dioxo cholan 24-oïque.

La présente invention a également pour objet un procédé de préparation des produits de formule générale I'

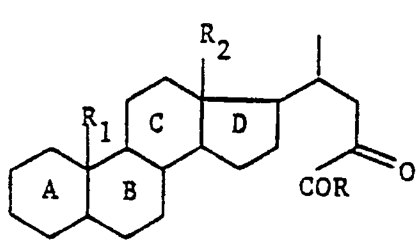

(I')

dans laquelle $R_1$ représente un atome d'hydrogène ou un radical méthyle, $R_2$ représente un radical méthyle ou éthyle, les noyaux A, B, C, D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkoxy renfermant de 1 à 4 atomes de carbone R représente un atome d'halogène, un radical hydroxyle, un radical alkoxy ayant de 1 à 6 atomes de carbone, un radical aralkoxy ayant de 7 à 15 atomes de carbone, un radical:

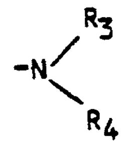

dans lequel $R_3$ et $R_4$, identiques ou différents représentent un atome d'hydrogène, un radical alkyle ayant de 1 à 6 atomes de carbone, un radical aralkyle ayant de 7 à 15 atomes de carbone ou $R_3$ et $R_4$ forment ensemble avec l'atome d'azote auquel ils sont liés un hétérocycle comportant éventuellement un autre hétéroatome choisi parmi les atomes d'azote et d'oxygène, ou R représente un radical alkylthio ayant de 1 à 6 atomes de carbone, un radical arylthio ou aralkylthio ayant au plus 15 atomes de carbone. à l'exception du produit dans lequel $R_1$ et $R_2$ représentent chacun un radical méthyl, R représente un radical méthoxy le noyau A porte une position 3-béta acétoxy et le noyau B porte une double liaison 5(6) caractérisé en ce que l'on traite un produit de formule II:

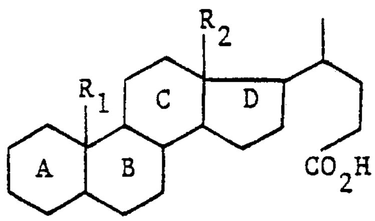

(II)

dans laquelle A, B, C, D, $R_1$ et $R_2$ ont la signification indiquée ci-dessus d'abord par un agent de formation d'un halogénure d'acide, ensuite par une base tertiaire puis par le chlorure de thionyle et enfin éventuellement par l'eau, un alcanol, un aralcanol, une amine primaire ou secondaire de formule:

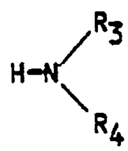

dans laquelle $R_3$ et $R_4$ ont la signification indiquée ci-dessus un alkylthiol, un arylthiol ou un aralkylthiol pour obtenir un produit de formule III:

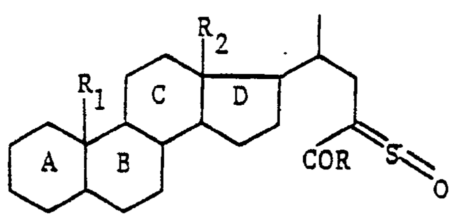

(III)

dans laquelle A, B C, D, R, $R_1$ et $R_2$ ont la signification indiquée ci-dessus, produit de formule III que l'on traite:

- soit: par un acide aqueux ou un oxydant.
- soit: par un réactif d'halogénation puis par un agent d'hydrolyse basique et traite éventuellement un produit de formule I obtenu dans lequel R représente un radical hydroxyle ou bien par un alcanol, un aralcanol une amine primaire ou secondaire de formule:

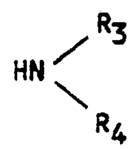

dans laquelle $R_3$ et $R_4$ ont la signification indiquée ci-dessus, un alkylthiol, un arylthiol ou un ou aralkylthiol, ou bien traite un produit de formule I obtenu dans laquelle R représente un radical hydroxyle par un agent de formation d'un halogénure d'acide.

Dans un mode préféré d'exécution du procédé ci-dessus, l'agent de formation d'un halogénure d'acide que l'on utilise de préférence est choisi parmi le chlorure de thionyle, le chlorure ou le bromure d'oxalyle. de préférence le chlorure de thionyle.

La base tertiaire que l'on utilise est choisie parmi la triéthylamine, la méthyl éthyl pyridine, la pyridine, le diazabicyclooctane, le diazabicylononène, le diazabicycloundécène, de préférence la triéthylamine ou la pyridine.

L'alcanol ou l'aralcanol que l'on utilise de préférence est le méthanol, l'éthanol ou l'alcool benzylique.

L'amine primaire ou secondaire que l'on peut utiliser est choisie parmi la métyl ou éthylamine, la diéthylamine,la morpholine, la pipéridine, la pyrrolidine, de préférence la morpholine. On peut bien entendu utiliser un thiol correspondant.

L'alkylthiol, l'arylthiol ou l'aralkylthiol que l'on peut utiliser est choisi de préférence, parmi le méthanethiol, l'éthanethiol et l'alcool thiobenzylique.

L'acide aqueux que l'on utilise de préférence pour transformer les produits de formule III en produits de formule I est l'acide sulfurique. On peut également utiliser un autre acide minéral ou organique, tel que l'acide chlorhydrique ou acétique.

Lorsque l'on utilise un oxydant, il peut s'agir du permanganate de potassium, de l'eau oxygénée, de l'ozone, d'un perborate ou d'un persulfate.

De manière générale, l'action d'un acide aqueux ou d'un oxydant sur les produits de formule III conduisent à un produit de formule I dans laquelle R représente un radical hydroxyle. Si nécessaire et si désiré, on peut alors préparer les autres produits de formule I en faisant agir de façon séparée ou simultanément, par exemple un alcanol, tel que le méthanol, pour otenir un produit de formule I dans laquelle R é alcoxy ou une amine primaire ou secondaire telle que la morpholine pour obtenir les produits de formule I dans laquelle

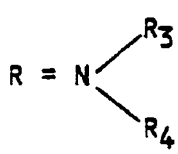

ainsi que les thiols correspondants. De manière préférentielle, on effectue l'hydrolyse acide ou l'oxydation en présence de l'alcanol ou de l'amine primaire ou secondaire dont on veut obtenir le dérivé de formule I.

On peut également, bien entendu, faire agir sur un produit de formule I dans lequel R = OH, un agent de formation d'un halogénure d'acide choisi dans la liste indiquée ci-dessus.

Les réactions énoncées ci-dessus pour la préparation de produits de formule III peuvent de préférence être effectuées dans un solvant ou un mélange de solvants peu ou non miscibles à l'eau tels que le chlorure de méthylène, le chloroforme.

Bien entendu, les réactions classiques de blocage ou de déblocage des groupements fonctionnels que peuvent comporter les cycles A, B, C et D peuvent être effectuées au départ de la synthèse, sur les produits de formule II ou sur les produits de formule 1 obtenus. On peut par exemple soumettre les produits de formule I dans laquelle le cycle A comporte en position 3, un radical hydroxyle protégé par un radical acyle tel que acétyle ou formyle à une réaction classique de saponification pour obtenir le produit

correspondant dans lequel le cycle A comporte un radical hydroxyle libre. On opère selon les méthodes usuelles par action, par exemple, d'une base telle que la soude, la potasse ou le carbonate de potassium dans un solvant tel que le méthanol, le chlorure de méthylène, l'eau ou un mélange de tels solvants.

On peut également inversement soumettre les produits de formule I à l'action d'un dérivé d'un groupement protecteur tel que l'anhydride acétique pour protéger un radical hydroxyle libre par exemple en position 3.

La présente invention a également pour objet une application des produits de formule I telle que définie ci-dessus à la préparation des produits de formule VI:

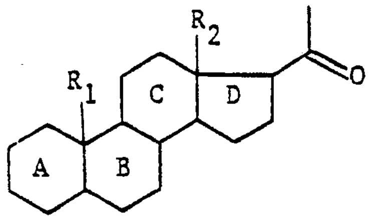

(VI)

dans laquelle A, B, C, D, $R_1$ et $R_2$ ont la signification indiquée ci-dessus caractérisée en ce que l'on traite un produit de formule I par un agent oxydant fort pour obtenir un produit de formule IV:

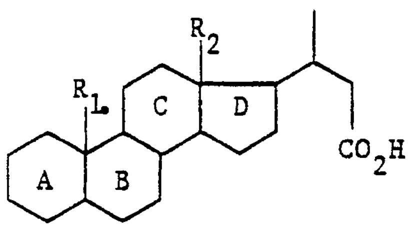

(IV)

dans laquelle A, B, C, D, $R_1$ et $R_2$ ont la signification indiquée ci-dessus. produit de formule IV que l'on traite d'abord par un agent de formation d'un halogénure d'acide, ensuite par une base tertiaire, puis par le chlorure de thionyle et par un agent d'halogénation et enfin éventuellement par l'eau, un alcanol, un aralcanol, une amine primaire ou secondaire de formule :

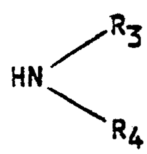

dans laquelle $R_3$ et $R_4$ ont la signification indiquée ci-dessus un alkylthiol, un arylthiol ou un aralkylthiol pour obtenir un produit de formule V:

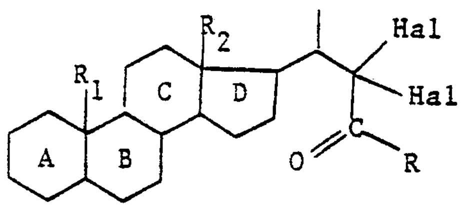

(V)

dans laquelle A, B, C, D, R, $R_1$, $R_2$ ont la signification indiquée ci-dessus et Hal représente un atome d'halogène. produit de formule V que l'on traite d'abord par un agent de déhydrohalogénation, puis par un agent de coupure oxydante pour obtenir un produit de formule VI attendu.

L'agent oxydant fort que l'on utilise peut être choisi dans la liste suivante : réactif de Jones (acides chromique et sulfurique dans l'eau) tétracétate de plomb, eau oxygénée dichromate de potassium.

L'agent de formation d'un halogénure d'acide que l'on utilise sur le produit de formule IV est choisi dans la liste indiquée ci-dessus, de préférence le chlorure de thionyle. Les autres réactifs sont également choisis dans les listes ci-dessus.

Le réactif d'halogénation que l'on utilise est un halogène tel que le brome ou un réactif d'halogénation tel que le chlorure de sulfuryle.

L'agent de déshydrohalogénation que l'on fait agir sur le produit de formule V est de préférence un agent basique fort tel que le triton B

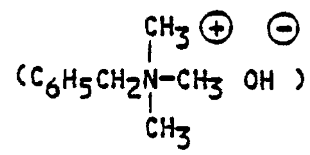

un alcoolate de métal alcalin tel que l'éthanolate de sodium ou de potassium, le tert-butylate de potassium, un amidure de sodium ou de potassium. On pourrait également envisager d'utiliser une base telle que la soude ou la potasse au reflux dans un alcanol tel que le méthanol ou l'éthanol, ou le glyme. On peut enfin envisager d'utiliser une résine basique telle que l'Amberlite.

L'agent de coupure oxydante que l'on peut utiliser pour obtenir en fin de synthèse les produits de formule VI attendus est choisi parmi l'ozone et un oxydant tel que l'oxyde de ruthénium ou l'oxyde de manganèse.

D'après les constatations de la demanderesse. l'action d'un réactif de déshydrohalogénation sur les produits de formule V donne naissance à un produit répondant à la formule $V_1$ suivante :

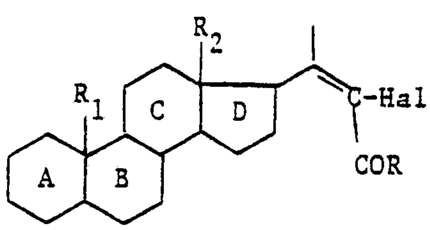
($V_1$)

produit qui est ensuite susceptible de conduire, après coupure oxydante aux produits de formule VI.

Bien entendu, dans l'application des produits de formule I à la préparation des produits de formule VI, les réactions classiques de blocage et de déblocage des groupements fonctionnels que peuvent comporter les cycles A, B, C et D peuvent être effectuées soit sur les produits de départ de formule I, soit sur des produits intermédiaires de la synthèse.

En particulier, dans le cas où la réaction de déshydrohalogénation effectuée sur les produits intermédiaires de formule V conduit à une saponification du groupement protecteur acyle tel qu'acétyle ou formyle. on peut réacyler le produit comportant un radical hydroxyle libre à l'aide par exemple d'anhydride acétique en présence de pyridine.

L'invention a également pour objet les procédés décrits ci-dessus caractérisés en ce que l'agent de formation d'un halogénure d'acide que l'on utilise est le chlorure de thionyle.

Comme la réaction de formation de la fonction sulfine comporte de toute façon l'utilisation du chlorure de thionyle, l'enchaînement des réactifs indiqué ci-dessus, à savoir :

a/. action d'un agent de formation d'un halogénure d'acide,

b/. base tertiaire, puis

c/. chlorure de thionyle, se résume, dans la forme préférée où l'agent de formation d'un halogénure d'acide est le chlorure de thionyle, à l'action, sur le produit de formule II du chlorure de thionyle en présence d'une base tertiaire.

L'invention a enfin pour objet, à titre de produits industriels nouveaux et notamment à titre de produits industriels nécessaires pour l'utilisation des produits de formule I les produits de formule telles que définies

ci-dessus.

Les produits de formule II utilisés au départ du procédé de préparation des produits de formule I sont des produits connus, pour beaucoup des produits naturels de la série des acides biliaires, ou des produits qui peuvent être préparés par les méthodes usuelles à partir de ces produits naturels.

Les produits de formule VI sont des produits de la série de la progestérone. Ces produits peuvent posséder des propriétés pharmacologiques intéressantes. Par ailleurs, ces produits peuvent servir de matière de base pour la reconstruction de la chaîne désoxycortisone :

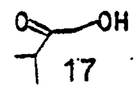

ou pour d'autres chaînes en position 17.

Les exemples suivants illustrent l'invention sans toutefois la limiter.

**Exemple 1 : Acide (3 alpha, 5 béta)-11,23-dioxo 3-hydroxy cholan 24-oïque.**

**Stade A** : Acide (3 alpha, 5 béta)-3-(acétyloxy) 11-oxo cholan 24-oïque.

On mélange 200 g d'acide 3 alpha-hydroxy 11-oxo 5 béta-cholane 24-oïque, 400 cm3 d'anhydride acétique, chauffe le mélange à 45¤C, introduit en une fois 2 g d'acide paratoluène sulfanique et 20 cm3 d'acide acétique, la température s'élève vers 63¤C en 5 minutes, on maintient pendant 1 heure à 60¤C, refroidit à 55¤C, introduit en 1 heure environ 400 cm3 d'eau distillée à +55¤C, refroidit à +10¤C, essore le précipité formé, le lave, le sèche sous pression réduite et obtient 211 g de produit attendu, F = 225¤C (pureté voisine de 99¤C).

On solubilise dans le chlorure de méthylène 106 g de produit obtenu, filtre sur silice, en éluant avec un mélange de chlorure de méthylène et d'acétate d'éthyle (9/1), et obtient 105 g de produit purifié F = 225¤C.

Contrôle :

Spectre IR (chloroforme) en cm-1

OH acide

1720 (ep)

C=O complexe

1705 (max)

C-O- acétate 1251

Spectre de RMN ($CDCl_3$) en ppm

| | | | |
|---|---|---|---|
| H de $CH_3$ en 18 | 0,62 | H de ACO | 2,03 |
| H de $CH_3$ en 21 | 0,88-0,93 | H en 3 | 4,72 |
| H de $CH_3$ en 19 | 1,2 | H de COOH | 8,1 |

**Stade B :** (3 alpha, 5 béta)-4-[3-(acétyloxy) 11,23,24-trioxo-cholan 24-yl] morpholine

On mélange, sous atmosphère inerte, 68 g d'acide (3 alpha, 5 béta) 3-(acétyloxy) 11-oxo-cholan 24-oïque, 250 cm3 de chlorure de méthylène, 0,35 cm3 de N, N-diméthyl formamide et ajoute au reflux du

chlorure de méthylène, en 15 minutes environ, 12,8 cm3 de chlorure de thionyle. On maintient au reflux pendant 45 minutes, concentre à sec par distillation sous pression réduite, ajoute au chlorure d'acide, cristallise 250 cm3 de chlorure de méthylène et introduit à -15¤C 12,8 cm3 de chlorure de thionyle. On ajoute à -25¤C en 1 heure et 30 minutes environ, un mélange de 46,5 cm3 de triéthylamine et de 46,5 cm3 de chlorure de méthylène, agite la suspension obtenue à pendant 30 minutes, ajoute en maintenant la température à -25¤C en 30 minutes environ un mélange de 35,5 cm3 de morpholine et de 50 cm3 de chlorure de méthylène, agite pendant 30 minutes, ajoute en 10 minutes environ 350 cm3 d'eau en laissant remonter la température vers 0¤C. On ajoute 4,7 cm3 d'acide acétique, ajoute à +2¤/+5¤C en 1 heure 30 minutes environ 49,6 g de permanganate de potassium et dilue au cours de cette introduction par 240 cm3 d'eau et agite à +2¤C/+5¤C pendant 1 heure. On ajoute à +5¤/+10¤C en 30 minutes environ 43 g de bisulfite de sodium et simultanément une solution de 12 cm3 d'acide sulfurique concentré dans 150 cm3 d'eau glacée. On décante, lave la phase chlorométhylinique à l'eau, sèche, ajoute 5 g de sulfate de magnésium, puis 60 g d'alumine $CBT_1$ sous bonne agitation, à 20¤C, en environ 1 heure et 30 minutes. On agite encore 1 heure 30 minutes à température ambiante, filtre, concentre le filtrat à sec par distillation sous pression réduite. On ajoute au résidu 80 cm3 d'acétate d'éthyle, concentre à sec par distillation sous pression réduite pour chasser le chlorure de méthylène résiduel et ajoute au résidu 100 cm3 d'éthanol. On solubilise sous agitation vers 40¤C, refroidit à 0¤C, amorce la cristallisation, laisse au repos pendant 16 heures et isole 57,6 g de produit attendu. F = 122-123¤C.

Les liqueurs mères sont concentrées à sec, on obtient un résidu de 22 g titrant 83,5% en produit attendu.

Contrôle :

Spectre IR (chloroforme) en $cm^{-1}$

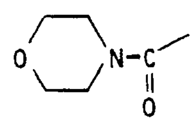 1641

région 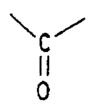 1723 (ep)

1715

1704

Spectre de RMN ($CDCl_3$) en ppm

| | |
|---|---|
| H de $CH_3$ en 18 | 0,67 |
| H de $CH_3$ en 21 | 0,9-1,0 |
| H de $CH_3$ en 19 | 1,17 |
| H de ACO | 2,0 |
| H en 3 | 4,7 |
| H de la morpholine | 3,4-3,8 |

**Stade C :** Acide (3 alpha, 5 béta)-11,23-dioxo 3-hydroxy cholan 24-oïque.

On mélange, sous atmosphère inerte 0,5 g de produit obtenu au stade précédent, 5 cm3 de méthanol à 5 % d'eau, 0,75 g de soude en pastilles et laisse 24 heures à température ambiante, la solution devient limpide, on ajoute une solution aqueuse 2 N d'acide chlorhydrique jusqu'à pH acide, extrait à l'acétate d'éthyle, sèche, concentre à sec par distillation sous pression réduite et obtient 0,4 g de produit attendu.

Contrôle :

Spectre IR (chloroforme) en $cm^{-1}$

| | | | | |
|---|---|---|---|---|
| 3 hydroxy OH | 3605 | | Région C=O | 1781 |
| 3 formes d'acide | 3410 | | ep | 1750 |
| | 1781 | | | 1700 |
| | 3510 | monomère | | 1710 |
| | | | | 1703 |

**Exemple 2 : Acide (3 alpha, 5 béta)-11-23-dioxo 3-hydroxy cholan 24-oïque.**

**Stade A :** Acide 3 alpha-formyl-oxy 11-oxo 23-sulfinyl 5 béta-cholan 24-oïque.

On mélange 83,7 g d'acide 3 alpha-formyloxy 11-oxo 5 béta-cholan 24-oïque, 840 cm3 de chlorure de méthylène, 168 cm3 de pyridine, refroidit à +10¤C, introduit en 5 minutes environ en laissant la température remonter à +20¤C 32 cm3 de chlorure de thionyle, agite à 20¤C pendant 1 heure, introduit en 5 minutes environ à 0¤C 84 cm3 d'eau, agite à 20¤C pendant 15 minutes, verse le mélange réactionnel dans une solution aqueuse glacée d'acide chlorhydrique, agite, décante, extrait au chlorure de méthylène, traite au charbon actif, concentre à sec par distillation sous pression réduite et obtient 98,5 g de produit attendu brut Rf. = 0,45, en éluant avec un mélange de chloroforme d'isopropanol et d'acide acétique (85/14/1).

Contrôle :

Analyse : $C_{25}H_{36}O_6S$ (464,60)

| | | | |
|---|---|---|---|
| Calculé : | C% 64,82 | H% 7,81 | S% 6,90 |
| Trouvé : | 64,8 | 7,7 | 7,0 |

Spectre UV (éthanol)

Max à 282 nm $E^1_1$ = 157 $\varepsilon$ = 6400

soit 77 % en sulfine.

**Stade B :** Ester méthylique de l'acide 3 alpha-hydroxy 11,23-dioxo 5-béta-cholan 24-oïque.

On mélange, sous atmosphère inerte, 2 g d'acide 3 alpha-formyloxy 11-oxo 23-sulfinyl 5 béta-cholan 24-oïque brut obtenu selon le stade A 20 cm3 de méthanol, 0,4 cm3 d'acide sulfurique concentré, chauffe pendant 2 heures à reflux, verse dans un mélange d'eau et de glace, extrait à l'acétate d'éthyle, lave à l'eau, sèche, concentre à sec par distillation sous pression réduite, chromatographie le résidu sur silice en éluant avec un mélange de chlorure de méthylène et d'acétate d'éthyle (9/1), et obtient 0,4 g de produit attendu. On cristallise dans un mélange de chlorure de méthylène et d'éther isopropylique et obtient le produit attendu purifié, F ≃ 75¤C (Rf. = 0,32 en élu nt par un mélange de chlorure de méthylène et d'acétate d'éthyle (85/15).

Spectre IR (chloroforme)

Absence de formiate - Présence de OH, cétone non conjuguée et bande ester large.

| | | |
|---|---|---|
| H de 18 Me | : | 0,67 |
| H de 20 Me | : | 0,9-1,0 |
| H de 19 Me | : | 1,14 |
| $H_3$ | : | 3,67 |
| H de $COOCH_3$ | : | 3,9 |

**Stade C :** Acide (3 alpha, 5 béta)-11,23-dioxo 3-hydroxy-cholan 24-oïque.

On mélange, sous atmosphère inerte, 0,5 g de produit obtenu au stade précédent, 5 cm3 de méthanol, 1,3 cm3 d'eau, 135 mg d'hydroxyde de sodium en pastilles, agite à 20¤C pendant 20 heures, verse le mélange réactionnel dans un mélange d'acide chlorhydrique N et de glace, agite, essore le précipité formé, le lave à l'eau, le dissout dans le chlorure de méthylène, sèche, concentre à sec par distillation sous pression réduite et obtient 0,43 g de produit attendu.
Contrôle :

Spectre IR (chloroforme) en cm-1

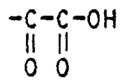 avec OH à 3412

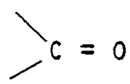 1781, 1722

autres 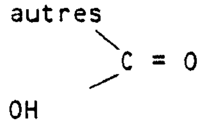 1702, 3602

**Exemple 3 : Acide alpha-hydroxy 11,23-dioxo 5 béta-cholan 24-oïque.**
**Stade A :** Acide 3 alpha-formyloxy 11-oxo 22,23-dibromo 5 béta-cholan 24-oïque.

On mélange, sous atmosphère inerte, 20,9 g d'acide 3 alpha formyloxy 11-oxo 5 béta cholan 24-oïque, 200 cm3 de chlorure de méthylène, 32 cm3 de pyridine. On introduit à +5¤C en 5 minutes environ en laissant la température remonter à +20¤C, 8 cm3 de chlorure de thionyle, agite à 20¤C pendant 1 heure, refroidit à 10¤C, introduit en 5 minutes environ 8 cm3 de brome et agite à 20¤C pendant 1 heure. On verse le mélange réactionnel dans un mélange d'eau et de glace, agite, décante, extrait au chlorure de méthylène, sèche, traite sur charbon actif avec un peu d'alumine, filtre, concentre à sec par distillation sous pression réduite, ajoute au résidu 40 cm3 d'acide formique, chauffe pendant 5 minutes à l'ébullition, élimine l'acide formique par distillation sous pression réduite. On ajoute lentement 40 cm3 d'éther isopropylique, glace, isole, 24,6 g de produit attendu. F = 248¤C, Rf. = 0,40 en éluant par un mélange de chloroforme, d'isopropanol, d'acide acétique (85/14/1).

| | | | | |
|---|---|---|---|---|
| Analyse | : | $C_{25}H_{36}O_5Br_2$ (576,38) | | |
| Calculé | : | C% 52,09 | H% 6,30 | Br% 27,73 |
| Trouvé | : | 52,0 | 6,3 | 27,4 |

**Stade B :** Acide 3 alpha hydroxy 11,23-dioxo 5 béta-cholane 24-oïque.

On mélange, sous atmosphère inerte, 46,6 g de produit obtenu comme au stade précédent, 930 cm3 de soude N , chauffe la suspension à 100¤C, pendant 4 heures, refroidit, ajoute de la glace, puis 100 cm3 d'acide chlorhydrique, concentre, extrait à l'acétate d'éthyle, sèche, traite au charbon actif avec un peu d'alumine, filtre, concentre à sec par distillation sous pression réduite. On ajoute du chlorure de méthylène au résidu, il y a cristallisation. On isole 25,4 g de produit attendu F = 155¤C environ.

On chromatographie le produit sur silice en éluant avec un mélange de chloroforme, isopropanol/acide acétique (80/18,5/1,5), concentre les fractions intéressantes, ajoute de l'acétate d'éthyle, lave à l'eau, sèche, concentre à sec par distillation sous pression réduite, cristallise dans un mélange d'acétone et d'éther de pétrole eb = 60-80¤C et obtient le produit attendu F = 130¤C, Rf = 0,35 en éluant avec un mélange de chloroforme 78/isopropanol 20/ acide acétique/2).

Spectre IR (chloroforme)
Présence de OH, 11-céto, et

$$-\overset{\displaystyle O}{\overset{\|}{C}}-COOH$$

Spectre de RMN ($CDCl_3$) en ppm

| | | |
|---|---|---|
| H de 18 Me | : | 0,68 |
| H de 20 Me | : | 0,9-1,0 |
| H de 19 Me | : | 1,18 |
| H mobiles | : | 4,4 |

**Exemple 4 : Acide (3 alpha, 5 béta) 3-(acétyloxy) 11,23-dioxo cholan 24-oïque.**

On mélange, sous atmosphère inerte, 141 mg d'acide (3 alpha, 5-béta) 11,23-dioxo 3-hydroxy cholan 24-oïque, 0,3 cm3 d'anhydride acétique, 3 mg d'acide paratoluène sulfonique, agite à 20¤C pendant 2 heures, ajoute 2 cm3 d'eau et agite pendant 15 minutes. On extrait à l'acétate d'éthyle, lave à l'eau, sèche, concentre à sec par distillation sous pression réduite, chromatographie le résidu sur silice en éluant par un mélange de chlorure de méthylène, d'isopropanol et d'acide acétique (87-12,5-0,5) et obtient 80 mg de produit attendu.

Spectre IR (chloroforme) en cm-1
présence de

$$\underset{\displaystyle O}{\underset{\|}{C}}-COOH$$

| | |
|---|---|
| OH acide | 3420 |
| >C = O | 1781<br>1723 |
| 11 céto | 1723 |

**Exemple 5 : 3 alpha-acétoxy 11,20-dioxo 5 béta-prégnane.**

**Stade A :** Acide (3 alpha, 5 béta) 3-(acétyloxy) 11-oxo 24-nor-cholan 23-oïque.

On mélange, sous atmosphère inerte, 893 mg d'acide (3 alpha, 5 béta) 3-(acétyloxy) 11,23-dioxo cholan 24-oïque 6 cm3 d'acide acétique, introduit à +15¤C, en 20 minutes environ 4,5 cm3 de solution oxydante de Jones, (préparée à partir de 267 g de $Cr_{O3}$, 230 cm3 de $H_2SO_4$, eau qsp 1000 cm3) agite à +15¤C pendant 5 minutes, verse le mélange réactionnel dans l'eau glacée. On extrait au chlorure de méthylène, lave avec une solution aqueuse 0,1M de thiosulfate de sodium, à l'eau, sèche et concentre à sec par distillation sous pression réduite. On chromatographie le résidu sur silice en éluant avec un mélange de chlorure de méthylène et d'acétone 9/1 et obtient 680 mg de produit attend F é 110-120¤C, peu net.
Spectre IR (chloroforme)
présence d'acétate, 11 oxo et acide

Spectre de RMN ($CDCl_3$) en ppm

| | |
|---|---|
| H de 18 Me | 0,66 |
| H de 21 Me | 0,97-1,02 |
| H de 19 Me | 1,18 |
| H de ACO | 2,0 |
| $H_3$ | 4,68 |

**Stade B :** 4-[(3 alpha, 5-béta) [3-(acétyloxy) 22,22-dibromo 11-oxo 24-norcholan 23-oyl]] morpholine.

On mélange, sous atmosphère inerte, 4,1 g de produit obtenu au stade précédent, 41 cm3 de chlorure de méthylène, 6,35 cm3 de pyridine, ajoute à 0¤/+5¤C 1,57 cm3 de chlorure de thionyle, puis aussitôt 1,6 cm3 de brome. On agite à 20¤C pendant une heure. On ajoute à 0¤/-5¤C, en 15 minutes environ 8,5 cm3 de morpholine, agite pendant 1 Heure en laissant remonter la température à 20¤C. On verse le mélange réactionnel dans 400 cm3 d'acide chlorhydrique 2 N glacé et extrait au chloroforme. On lave à l'eau, sèche, concentre à sec par distillation sous pression réduite. On chromatographie le résidu (6,5 g) sur silice en éluant avec le mélange cyclohexane-acétate d'éthyle (8/2) et obtient 2,075 g de produit attendu.
Contrôle :

Spectre IR (chloroforme) en $cm^{-1}$

| | |
|---|---|
| OAC | 1724 |
| | 1364 |
| C-O complexe | 1267-1251-1235 |
| 11-oxo | 1704 |
| amide | 1645 |

Spectre de RMN ($CDCl_3$) en ppm

| | |
|---|---|
| H de 18 Me | 0,74 |
| H de 19 Me | 1,16 |
| H de 21 Me | 1,34-1,42 |
| H de ACO | 1,8 |
| H de la morpholine | 3,76 |
| $H_3$ | 4,7 |

Analyse : $C_{29}H_{43}Br_2NO_5$ (645,48)

| | | | | |
|---|---|---|---|---|
| Calculé : | C% 53,96 | H% 6,71 | N% 2,17 | Br% 24,76 |
| Trouvé : | 53,9 | 6,7 | 2,1 | 24,6 |

**Stade C :** 3 alpha acétoxy 11,20-dioxo 5 béta-prégnane.

1¤/. Débromhydratation

On mélange, sous atmosphère inerte 250 mg de produit obtenu au stade précédent, 2,5 cm3 de méthanol, 2,5 cm3 de triton B (ou hydroxyde de benzyl triméthyl ammonium) en solution aqueuse à 40 %, chauffe au reflux pendant 1 heure, refroidit, verse le mélange réactionnel dans l'eau glacée, extrait au chlorure de méthylène, lave à l'eau, sèche, concentre à sec par distillation sous pression réduite et obtient 120 mg de produit attendu utilisé tel quel pour la réaction suivante.

2¤/. Acétylation

On mélange sous atmosphère inerte le produit obtenu ci-dessus, 1,2 cm3 de pyridine, 0,48 cm3 d'anhydride acétique et laisse 20 heures en contact. On verse dans l'eau glacée, après 30 minutes, extrait au chlorure de méthylène. On lave à l'eau, sèche, concentre à sec par distillation sous pression réduite et obtient 170 mg de produit acétylé attendu utilisé tel quel pour la réaction suivante.

3¤/. Ozonolyse

On mélange, sous atmosphère inerte le produit obtenu, 2,5 cm3 de 1,2-dichloréthane, 1 cm3 d'acide acétique et fait passer à -5¤C un courant d'oxygène ozonisé pendant 15 minutes. On élimine l'excès d'ozone par un barbotage d'azote, verse le mélange réactionnel lentement dans un excès de solution aqueuse de bicarbonate de sodium. On extrait au chlorure de méthylène, lave à l'eau, sèche, concentre à sec par distillation sous pression réduite et obtient 115 mg de produit attendu (3 alpha-acétoxy 11,20-dioxo 5 béta-prégnane).

On chromatographie le produit brut sur silice en éluant avec un mélange de cyclohexane et d'acétate d'éthyle (8/2) et obtient 16 mg de produit attendu purifié dont le spectre infra-rouge est identique à celui d'un échantillon authentique.

**Exemple 6 : 3 alpha-acétoxy 11,20-dioxo 5 béta-prégnane à partir d'acide (3 alpha, 5 béta) 3-(acétyloxy) 11-oxo 24-norcholane 23-oïque.**

1¤/. Bromuration

On mélange, sous atmosphère inerte 3,2 g d'acide (3 apha 5 béta) 3-(acétyloxy) 11-oxo 24-nor cholan 23-oïque (titre 85%) tel qu'obtenu au stade A de l'exemple 5, 32 cm3 de chlorure de méthylène, 6,1 cm3 de pyridine, ajoute à -5¤C, goutte à goutte, en 10 minutes environ 1,2 cm3 de chlorure de thionyle, puis à -10¤C, en 10 minutes environ 0,6 cm3 de brome. On agite pendant 2 heures et 30 minutes à 20¤C introduit à -5¤C en 20 minutes environ 8 cm3 de diéthylamine, agite pendant 1 heure à 20¤C, verse le mélange réactionnel dans de l'acide chlorydrique 2N glacé.

On agite 15 minutes, extrait au chlorure de méthylène, lave à l'eau, sèche, traite au charbon actif et amène à sec par distillation sous pression réduite. On obtient 5,1 g de produit brut de bromuration attendu, utilisé tel quel pour la réaction suivante.

2¤/. Traitement au Triton B (Débromhydratation)

On mélange, sous atomosphère inerte, 1,5 g de produit brut de bromuration obtenu ci-dessus, 15 cm3 de méthanol, 12 cm3 de triton B (hydroxyde de benzyl triméthyl ammonium) en solution aqueuse à 40 %

et chauffe au reflux pendant 1 heure et 30 minutes.

On refroidit, verse dans l'eau, extrait au chlorure de méthylène, lave à l'eau saturée en chlorure de sodium avec une solution aqueuse 0,5 M de phosphate monosodique, à l'eau saturée en chlorure de sodium, sèche, concentre à sec par distillation sous pression réduite et obtient 837 mg de produit brut attendu utilisé tel quel pour la réaction suivante.

3¤/. Acétylation

On mélange, sous atmosphère inerte, les 837 mg de produit obtenu précédemment, 3 cm3 de pyridine, 1,5 cm3 d'anhydride acétique, agite à 20¤C pendant 20 heures, ajoute quelques cm3 d'eau, agite pendant 1 heure, verse le mélange réactionnel dans l'eau saturée en chlorure de sodium, extrait au chlorure de méthylène, lave à l'eau saturée en chlorure de sodium, sèche et concentre à sec par distillation sous pression réduite et obtient 954 mg de produit acétylé brut attendu. Ce produit est utilisé tel quel pour la réaction suivante.

4¤/. Ozonisation

On mélange les 954 mg de produit acétylé brut obtenu au paragraphe 3 ci-dessus, 15 cm3 de chlorure de méthylène, 8 cm3 d'acide acétique, fait passer à 0¤C un courant d'oxygène ozonisé pendant 1 heure, verse le mélange réactionnel dans l'eau, extrait au chlorure de méthylène, lave à l'eau saturée en chlorure de sodium, sèche, concentre à sec par distillation sous pression réduite et obtient 992 mg de produit ozonisé brut. On chromatographie le produit brut sur silice en éluant avec un mélange de cyclohexane et d'acétate d'éthyle (75/25) et obtient 106 mg de 3 alpha-acétoxy 11,20-dioxo 5 béta-prégnane attendu.

Spectre IR (chloroforme)

Spectre identique à celui d'un échantillon authentique de 3 alpha-acétoxy 11,20-dioxo 5 béta-prégnane.

## Revendications

**1.** Les produits de formule générale I:

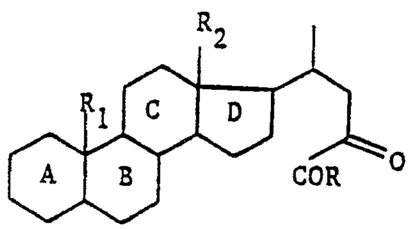

(I)

dans laquelle $R_1$ représente un atome d'hydrogène ou un radical méthyle, $R_2$ représente un radical méthyle ou éthyle, les noyaux A, B, C, D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkoxy renfermant de 1 à 4 atomes de carbone R représente un atome d'halogène, un radical hydroxyle, un radical alkoxy ayant de 1 à 6 atomes de carbone, un radical aralkoxy ayant de 7 à 15 atomes de carbone, un radical:

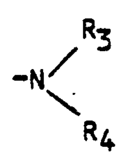

dans lequel $R_3$ et $R_4$, identiques ou différents représentent un atome d'hydrogène, un radical alkyle ayant de 1 à 6 atomes de carbone, un radical aralkyle ayant de 7 à 15 atomes de carbone ou $R_3$ et $R_4$ forment ensemble avec l'atome d'azote auquel ils sont liés un hétérocycle comportant éventuellement un autre hétéroatome choisi parmi les atomes d'azote et d'oxygène, ou R représente un radical alkylthio ayant de 1 à 6 atomes de carbone, un radical arylthio ou aralkylthio ayant au plus 15 atomes de carbone, à l'exception du produit dans lequel $R_1$ et $R_2$ représentent chacun un radical méthyle, R représente un radical méthoxy le noyau A porte une position 3-béta acétoxy et le noyau B porte une double liaison 5 (6) et étant entendu que le noyau C comporte soit une fonction cétonique éventuellement protégée en position 11 soit une fonction hydroxyle éventuellement protégée en position 12.

2. Les produits de formule générale I telle que définie à la revendication 1 dans laquelle $R_1$ et $R_2$ représentent chacun un radical méthyle et les noyaux A, B, C, D portent en position 3 une fonction hydroxyle éventuellement protégée et, éventuellement, une ou plusieurs autres fonctions choisies parmi les fonctions hydroxyles éventuellement protégées en position 6, 7 et 12 et les fonctions cétones éventuellement protégées en position 7 et 11 et R représente un radical hydroxy, un radical alkoxy ayant au plus 4 atomes de carbone ou un radical:

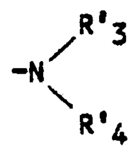

dans lequel $R'_3$ et $R'_4$ représentent un atome d'hydrogène, un radical alkyle ayant au plus 4 atomes de carbone ou $R'_3$ et $R'_4$ forment ensemble avec l'atome d'azote auquel ils sont liés un radical pipéridino, pyrrolidino ou morpholino.

3. Les produits de formule générale I telle que définie à l'une des revendications 1 ou 2 qui portent en position 3 un radical hydroxyle éventuellement protégé et R représente un radical hydroxyle, méthoxy ou éthoxy ou un radical morpholino.

4. Les produits de formule générale I telle que définie à l'une des revendications 1 à 3 répondant aux formules suivantes :
   - La (3 alpha, 5 béta) 4-3-(acétyloxy) 11,23, 24-trioxo-cholan 24-yl/morpholine.
   - L'acide (3 alpha, 5 béta) 11, 23-dioxo 3-hydroxy cholan 24-oïque.
   - L'ester méthylique de l'acide 3 alpha-hydroxy 11, 23-dioxo 5-béta-cholan 24-oïque.
   - L'acide (3 alpha, 5 béta) 3-(acétyloxy) 11, 23-dioxo cholan 24-oïque.

5. Procédé de préparation des produits de formule I'

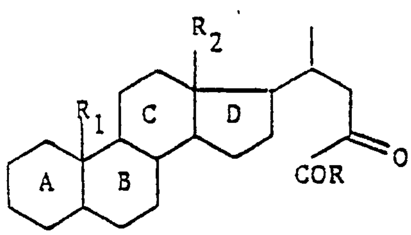

(I')

dans laquelle $R_1$ représente un atome d'hydrogène ou un radical méthyle. $R_2$ représente un radical méthyle ou éthyle, les noyaux A, B, C, D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkoxy renfermant de 1 à 4 atomes de carbone R représente un atome d'halogène, un radical hydroxyle, un radical alkoxy ayant de 1 à 6 atomes de carbone, un radical aralkoxy ayant de 7 à 15 atomes de carbone, un radical :

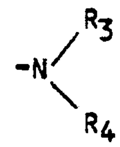

dans lequel $R_3$ et $R_4$, identiques ou différents représentent un atome d'hydrogène, un radical alkyle ayant de 1 à 6 atomes de carbone, un radical aralkyle ayant de 7 à 15 atomes de carbone ou $R_3$ et $R_4$ forment ensemble avec l'atome d'azote auquel ils sont liés un hétérocycle comportant éventuellement un autre hétéroatome choisi parmi les atomes d'azote et d'oxygène, ou R représente un radical alkylthio ayant de 1 à 6 atomes de carbone, un radical arylthio ou aralkylthio ayant au plus 15 atomes de carbone, à l'exception du produit dans lequel $R_1$ et $R_2$ représentent chacun un radical méthyle, R représente un radical méthoxy le noyau A porte une position 3-béta acétoxy et le noyau B porte une double liaison 5 (6), caractérisé en ce que l'on traite un produit de formule II :

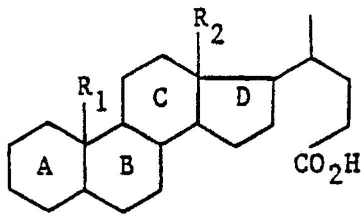

(II)

dans laquelle A, B, C, D, $R_1$ et $R_2$ ont la signification indiquée à la revendication 1 d'abord par un agent de formation d'un halogénure d'acide, ensuite par une base tertiaire puis par le chlorure de thionyle et enfin éventuellement par l'eau, un alcanol, un aralcanol, une amine primaire ou secondaire de formule :

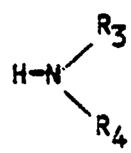

dans laquelle $R_3$ et $R_4$ ont la signification indiquée à la revendication 1, un alkylthiol, un arylthiol ou un aralkylthiol pour obtenir un produit de formule III :

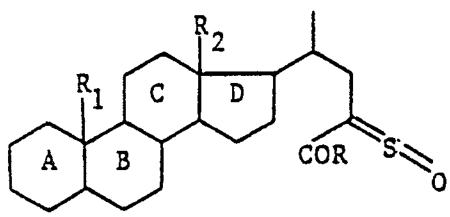

(III)

dans laquelle A, B, C, D, R, $R_1$ et $R_2$ ont la signification indiquée à la revendication 1, produit de formule III que l'on traite :

- soit : par un acide aqueux ou un oxydant,
- soit : par un réactif d'halogénation puis par un agent d'hydrolyse basique et traite éventuellement un produit de formule I' obtenu dans lequel R représente un radical hydroxyle ou bien par un alcanol, un aralcanol, une amine primaire ou secondaire de formule :

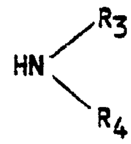

dans laquelle $R_3$ et $R_4$ ont la signification indiquée à la revendication 1, un alkylthiol, un arylthiol ou un aralkylthiol, ou bien traite un produit de formule I' obtenu dans lequel R représente un radical hydroxyle par un agent de formation d'un halogénure d'acide.

**6.** Application des produits de formule I à la préparation des produits de formule VI:

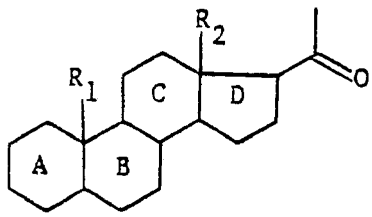

(VI)

dans laquelle A, B, C, D, $R_1$ et $R_2$ ont la signification indiquée à la revendication 1, caractérisée en ce que l'on traite un produit de formule I par un agent oxydant fort pour obtenir un produit de formule IV:

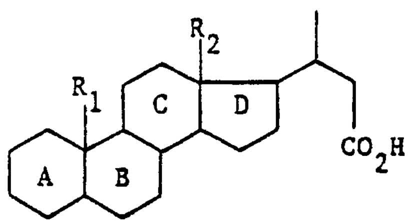

(IV)

dans laquelle A, B, C, D, $R_1$ et $R_2$ ont la signification indiquée à la revendication 5, produit de formule IV que l'on traite d'abord par un agent de formation d'un halogénure d'acide, ensuite par une base tertiaire, puis par le chlorure de thionyle et par un agent d'halogénation et enfin éventuellement par l'eau, un alcanol, un aralcanol, une amine primaire u secondaire de formule :

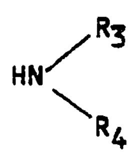

dans laquelle $R_3$ et $R_4$ ont la signification indiquée à la revendication 1, un alkylthiol, un arylthiol ou un aralkylthiol pour obtenir u produit de formule V:

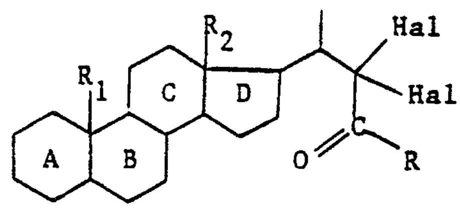

(V)

dans laquelle A, B, C, D, R, $R_1$, $R_2$ et R ont la signification indiquée à la revendication 1 et Hal représente un atome d'halogène, produit de formule V que l'on traite d'abord par un agent de déhydrohalogénation, puis par un agent de coupure oxydante pour obtenir un produit de formule VI attendu.

7. Procédé selon l'une quelconque des revendications 5 ou 6 caractérisés en ce que l'agent de formation d'un halogénure d'acide que l'on utilise est le chlorure de thionyle.

8. A titre de produits industriels nouveaux, les produits de formule V tels que définis à la revendication 6.

Revendications pour l'Etat contractant suivant : ES

1. Procédé de préparation des produits de formule générale I:

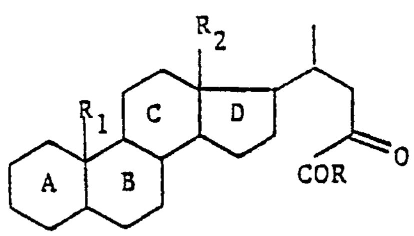

(I)

dans laquelle $R_1$ représente un atome d'hydrogène ou un radical méthyle, $R_2$ représente un radical méthyle ou éthyle, les noyaux A, B, C, D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkoxy renfermant de 1 à 4 atomes de carbone R représente un atome d'halogène, un radical hydroxyle, un radical alkoxy ayant de 1 à 6 atomes de carbone, un radical aralkoxy ayant de 7 à 15 atomes de carbone, un radical :

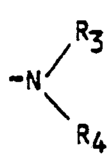

dans lequel $R_3$ et $R_4$, identiques ou différents représentent un atome d'hydrogène, un radical alkyle ayant de 1 à 6 atomes de carbone, un radical aralkyle ayant de 7 à 15 atomes de carbone ou $R_3$ et $R_4$ forment ensemble avec l'atome d'azote auquel ils sont liés un hétérocycle comportant éventuellement un autre hétéroatome choisi parmi les atomes d'azote et d'oxygène, ou R représente un radical alkylthio ayant de 1 à 6 atomes de carbone, un radical arylthio ou aralkylthio ayant au plus 15 atomes de carbone, à l'exception du produit dans, lequel $R_1$ et $R_2$ représentent chacun un radical méthyle, R represente un radical méthoxy le noyau A porte une fonction 3-béta acétoxy et le noyau B porte une double liaison 5 (6), caractérisé en ce que l'on traite un produit de formule II:

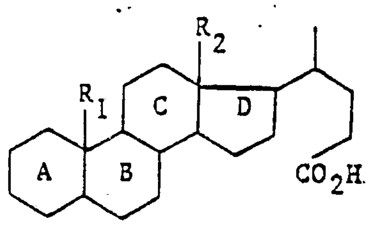

(II)

dans laquelle A, B, C, D, $R_1$ et $R_2$ ont la signification indiquée ci-dessus, d'abord par un agent de formation d'un halogénure d'acide, ensuite par une base tertiaire puis par le chlorure de thionyle et enfin éventuellement par l'eau, un alcanol, un aralcanol, une amine primaire ou secondaire de formule :

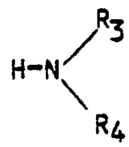

dans laquelle $R_3$ et $R_4$ ont la signification indiquée ci-dessus, un alkylthiol, un arylthiol ou un aralkylthiol pour obtenir un produit de formule III:

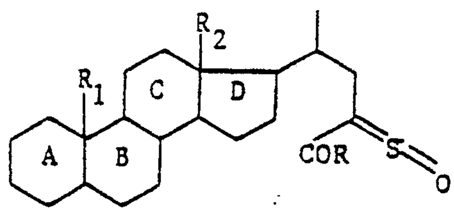

(III)

dans laquelle A, B, C, D R, $R_1$ et $R_2$ ont la signification indiquée ci-dessus, produit de formule III que l'on traite:

- soit : par un acide aqueux ou un oxydant,
- soit : par un réactif d'halogénation puis par un agent d'hydrolyse basique et traite éventuellement un produit de formule I obtenu dans lequel R représente un radical hydroxyle ou bien par un alcanol, un aralcanol, une amine primaire ou secondaire de formule :

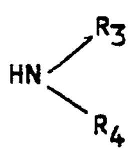

dans laquelle $R_3$ et $R_4$ ont la signification indiquée ci-dessus, un alkylthiol, un arylthiol ou un aralkylthiol, ou bien traite un produit de formule I obtenu dans lequel R représente un radical hydroxyle par un agent de formation d'un halogénure d'acide.

2. Procédé selon la revendication 1, caractérisé en ce que produit de formule (II) utilisé au départ et les réactifs sont choisis de manière telle que l'on prépare des produits de formule (1) dans laquelle $R_1$ et $R_2$ représentent chacun un radical méthyle et les noyaux A, B, C, D portent en position 3 une fonction hydroxyle éventuellement protégée et, éventuellement, une ou plusieurs autres fonctions choisies parmi les fonctions hydroxyles éventuellement protégées en position 6, 7, 11 et 12 et les fonctions cétones éventuellement protégées en position 7, 11 et 12 et R représente un radical hydroxy, un radical alkoxy ayant au plus 4 atomes de carbone ou un radical :

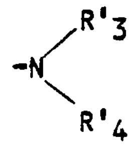

dans lequel R'$_3$ et R'$_4$ représentent un atome d'hydrogène, un radical alkyle ayant au plus 4 atomes de carbone ou R'$_3$ et R'$_4$ forment ensemble avec l'atome d'azote auquel ils sont liés un radical pipéridino, pyrrolidino ou morpholino.

3. Procédé selon la revendication 1 ou 2, caractérisé en ce que le produit de formule (II) utilisé au départ et les réactifs sont choisis de manière telle que l'on prépare des produits de formule (I), dans laquelle les noyaux A. B, C, D portent en position 3 un radical hydroxyle éventuellement protégé et éventuellement une ou plusieurs autres fonctions choisies parmi les fonctions hydroxyles éventuellement protégées en position 12 et les fonctions cétones éventuellement protégées en position 11 ou 12 et R représente un radical hydroxyle, méthoxy ou éthoxy ou un radical morpholino.

4. Application des produits de formule I à la préparation des produits de formule VI:

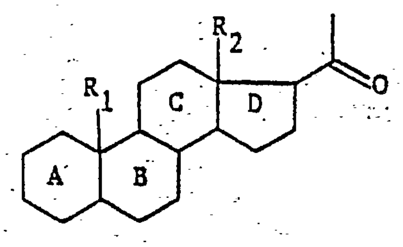

(VI)

dans laquelle A, B, C, D, R$_1$ et R$_2$ ont la signification indiquée à la revendication 1. caractérisée en ce que l'on traite un produit de formule I par un agent oxydant fort pour obtenir un produit de formule IV:

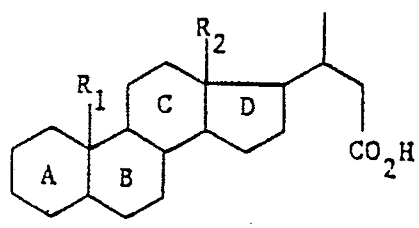

(IV)

dans laquelle A, B, C, D, R$_1$ et R$_2$ ont la signification indiquée à la revendication 1, produit de formule IV que l'on traite d'abord par un agent de formation d'un halogénure d'acide, ensuite par une base tertiaire, puis par le chlorure de thionyle et par un agent d'halogénation et enfin éventuellement par l'eau, un alcanol, un aralcanol, une amine primaire ou secondaire de formule :

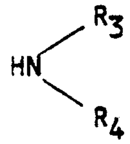

dans laquelle R$_3$ et R$_4$ ont la signification indiquée à la revendication 1, un alkylthiol, un arylthiol ou un aralkylthiol pour obtenir un produit de formule V:

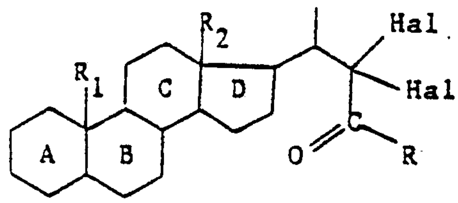

(V)

dans laquelle A, B, C, D, R, $R_1$, $R_2$ et R ont la signification indiquée à la revendication 1 et Hal représente un atome d'halogène produit de formule V que l'on traite d'abord par un agent de déhydrohalogénation, puis par un agent ce coupure oxydante pour obtenir un produit de formule VI attendu.

**5.** Procédé selon l'une quelconque des revendications 1 ou 4, caractérisés en ce que l'agent de formation d'un halogénure d'acide que l'on utilise est le chlorure de thionyle.

Revendications pour l'Etat contractant suivant : GR

**1.** Procédé de préparation des produits de formule générale I :

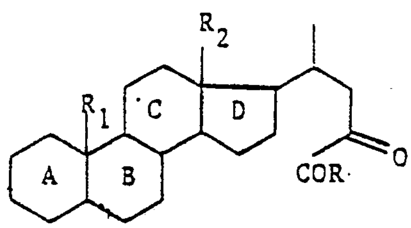

(I)

dans laquelle $R_1$ représente un atome d'hydrogène ou un radical méthyle, $R_2$ représente un radical méthyle ou éthyle, les noyaux A, B, C, D portent éventuellement une ou plusieurs doubles liaisons et sont éventuellement substitués par une ou plusieurs fonctions hydroxyle ou cétone éventuellement protégées, par un ou plusieurs atomes d'halogène, par un ou plusieurs radicaux alkyle ou alkoxy renfermant de 1 à 4 atomes de carbone R représente un atome d'halogène, un radical hydroxyle, un radical alkoxy ayant de 1 à 6 atomes de carbone, un radical aralkoxy ayant de 7 à 15 atomes de carbone, un radical :

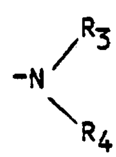

dans lequel $R_3$ et $R_4$, identiques ou différents représentent un atome d'hydrogène, un radical alkyle ayant de 1 à 6 atomes de carbone, un radical aralkyle ayant de 7 à 15 atomes de carbone ou $R_3$ et $R_4$ forment ensemble avec l'atome d'azote auquel ils sont liés un hétérocycle comportant éventuellement un autre hétéroatome choisi parmi les atomes d'azote et d'oxygène, ou R représente un radical alkylthio ayant de 1 à 6 atomes de carbone, un radical arylthio ou aralkylthio ayant au plus 15 atomes de carbone, à l'exception du produit dans lequel $R_1$ et $R_2$ représentent chacun un radical méthyle, R represente un radical méthoxy le noyau A porte une fonction 3-béta acétoxy et le noyau B porte une double liaison 5 (6), caractérisé en ce que l'on traite un produit de formule II:

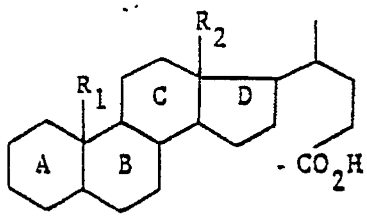

(II)

dans laquelle A, B, C, D, $R_1$ et $R_2$ ont la signification indiquée ci-dessus, d'abord par un agent de formation d'un halogénure d'acide, ensuite par une base tertiaire puis par le chlorure de thionyle et enfin éventuellement par l'eau, un alcanol, un aralcanol, une amine primaire ou secondaire de formule :

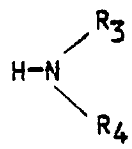

dans laquelle $R_3$ et $R_4$ ont la signification indiquée ci-dessus, un alkylthiol, un arylthiol ou un aralkylthiol pour obtenir un produit de formule III :

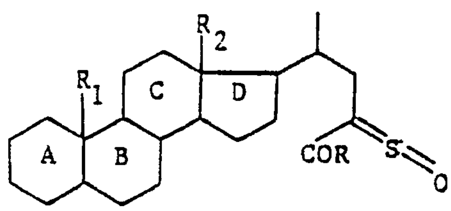

(III)

dans laquelle A, B, C, D, R, $R_1$ et $R_2$ ont la signification indiquée ci-dessus, produit de formule III que l'on traite :

- soit : par un acide aqueux ou un oxydant,
- soit : par un réactif d'halogénation puis par un agent d'hydrolyse basique et traite éventuellement un produit de formule I obtenu dans lequel R représente un radical hydroxyle ou bien par un alcanol, un aralcanol, une amine primaire ou secondaire de formule :

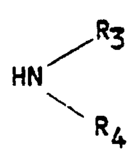

dans laquelle $R_3$ et $R_4$ ont la signification indiquée ci-dessus, un alkylthiol, un arylthiol ou un aralkylthiol, ou bien traite un produit de formule I obtenu dans lequel R représente un radical hydroxyle par un agent de formation d'un halogénure d'acide.

**2.** Procédé selon la revendication 1, caractérisé en ce que produit de formule (II) utilisé au départ et les réactifs sont choisis de manière telle que l'on prépare des produits de formule (I) dans laquelle $R_1$ et $R_2$ représentent chacun un radical méthyle et les noyaux A, B, C, D portent en position 3 une fonction hydroxyle éventuellement protégée et, éventuellement, une ou plusieurs autres fonctions choisies parmi les fonctions hydroxyles éventuellement protégées en position 6, 7, 11 et 12 et les fonctions cétones éventuellement protégées en position 7, 11 et 12 et R représente un radical hydroxy, un radical alkoxy ayant au plus 4 atomes de carbone ou un radical :

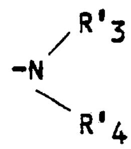

dans lequel $R'_3$ et $R'_4$ représentent un atome d'hydrogène, un radical alkyle ayant au plus 4 atomes de carbone ou R'3 et R'4 forment ensemble avec l'atome d'azote auquel ils sont liés un radical pipéridino; pyrrolidino ou morpholino.

**3.** Procédé selon la revendication 1 ou 2, caractérisé en ce que le produit de formule (II) utilisé au départ et les réactifs sont choisis de manière telle que l'on prépare des produits de formule (I), dans laquelle les noyaux A, B, C, D portent en position 3 un radical hydroxyle éventuellement protégé et éventuellement une ou plusieurs autres fonctions choisies parmi les fonctions hydroxyles éventuellement protégées en position 12 et les fonctions cétones éventuellement protégées en position 11 ou 12 et R représente un radical hydroxyle, méthoxy ou éthoxy ou un radical morpholinc.

**4.** Procédé selon l'une quelconque des revendications 1 à 3, caractérisé en ce que l'on prépare les produits de formule générale (I) dont les noms suivent :

- la (3 alpha, 5 béta) 4-3-(acétyloxy) 11, 23, 24-trioxo-cholan 24-yl/morpholine,
- l'acide (3 alpha, 5 béta) 11, 23-dioxo 3-hydroxy cholan 24-oïque,
- l'ester méthylique de l'acide 3 alpha-hydroxy 11, 23-dioxo 5-béta-cholan 24-oïque,
- l'acide (3 alpha, 5 béta) 3-(acétyloxy) 11, 23-dioxo cholan 24-oïque,

**5.** Application des produits de formule I à la préparation des produits de formule VI :

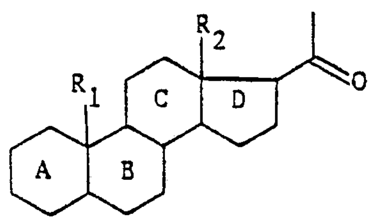

(VI)

dans laquelle A, B, C, D, $R_1$ et $R_2$ ont la signification indiquée à la revendication 1, caractérisée en ce que l'on traite un produit de formule I par un agent oxydant fort pour obtenir un produit de formule IV :

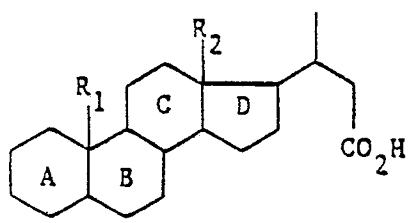

(IV)

dans laquelle A, B, C, D, $R_1$ et $R_2$ ont la signification indiquée à la revendication 1, produit de formule IV que l'on traite d'abord par un agent de formation d'un halogénure d'acide, ensuite par une base tertiaire, puis par le chlorure de thionyle et par un agent d'halogénation et enfin éventuellement par l'eau, un alcanol, un aralcanol, une amine primaire ou secondaire de formule :

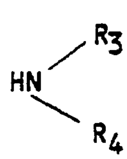

dans laquelle $R_3$ et $R_4$ ont la signification indiquée à la revendication 1, un alkylthiol, un arylthiol ou un aralkylthiol pour obtenir un produit de formule V :

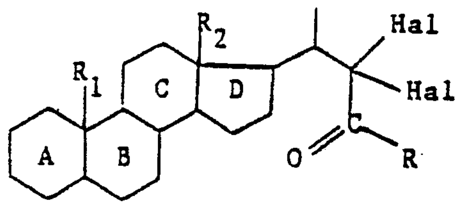

(V)

dans laquelle A. B, C, D, R, $R_1$, $R_2$ et R ont la signification indiquée à la revendication 1 et Hal représente un atome d'halogène, produit de formule V que l'on traite d'abord par un agent de déhydrohalogénation, puis par un agent de coupure oxydante pour obtenir un produit de formule VI attendu.

6. Procédé selon l'une quelconque des revendications 1 ou 5, caractérisés en ce que l'agent de formation d'un halogénure d'acide que l'on utilise est le chlorure de thionyle.

7. A titre de produits industriels nouveaux, les produits de formules V tels que définis à la revendication 5.

**Claims**

1. The products of general formula (I):

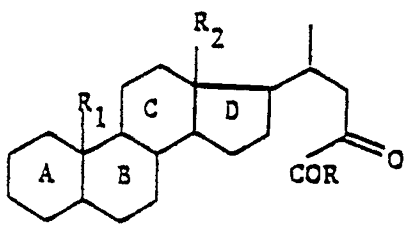

(I)

in which $R_1$ represents a hydrogen atom or a methyl radical, $R_2$ represents a methyl or ethyl radical, the nuclei A, B, C, D optionally carry one or more double bonds and are optionally substituted by one or more optionally protected hydroxyl or ketone functions, by one or more halogen atoms, by one or more alkyl or alkoxy radicals containing 1 to 4 carbon atoms, R represents a halogen atom, a hydroxyl radical, an alkoxy radical having 1 to 6 carbon atoms, an aralkoxy radical having 7 to 15 carbon atoms, a radical:

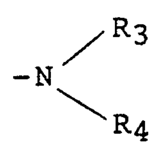

in which $R_3$ and $R_4$, identical or different, represent a hydrogen atom, an alkyl radical having 1 to 6 carbon atoms, an aralkyl radical having 7 to 15 carbon atoms or $R_3$ and $R_4$ form together with the nitrogen atom to which they are linked a heterocycle optionally containing another heteroatom chosen from nitrogen and oxygen atoms, or R represents an alkylthio radical having 1 to 6 carbon atoms, an arylthio or aralkylthio radical having at most 15 carbon atoms, with the exception of the product in which $R_1$ and $R_2$ each represents a methyl radical, R represents a methoxy radical, the A nucleus carries a 3-beta acetoxy position and the B nucleus carries a double bond 5(6), and it being understood that the C nucleus contains either a ketone function optionally protected in position 11 or a hydroxyl

function optionally protected in position 12.

2. The products of general formula (I) as defined in claim 1 in which $R_1$ and $R_2$ each represents a methyl radical and the nuclei A, B, C, D carry in position 3 an optionally protected hydroxyl function and, optionally, one or more other functions chosen from the hydroxyl functions optionally protected in position 6, 7 and 12 and the ketone functions optionally protected in position 7 and 11 and R represents a hydroxy radical, an alkoxy radical having at most 4 carbon atoms or a radical:

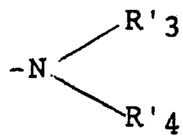

in which $R'_3$ and $R'_4$ represent a hydrogen atom, an alkyl radical having at most 4 carbon atoms or $R'_3$ and $R'_4$ form together with the nitrogen atom to which they are linked a piperidino, pyrrolidino or morpholino radical.

3. The products of general formula (I) as defined in one of claims 1 or 2 which carry in position 3 an optionally protected hydroxyl radical and in which R represents a hydroxyl, methoxy or ethoxy radical or a morpholino radical.

4. The products of general formula (I) as defined in one of claims 1 to 3 corresponding to the following formulae:
- (3-alpha, 5-beta)-4-3-(acetyloxy)-11,23,24-trioxo-cholan-24-yl morpholine.
- (3-alpha, 5-beta)-11,23-dioxo-3-hydroxy-cholan-24-oic acid.
- Methyl ester of 3-alpha-hydroxy-11,23-dioxo-5-beta-cholan-24-oic acid.
- (3-alpha, 5-beta)-3-(acetyloxy)-11,23-dioxo-cholan-24-oic acid.

5. Preparation process for products of formula (I'):

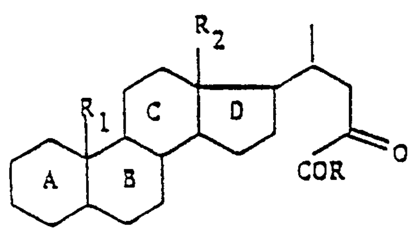

(I')

in which $R_1$ represents a hydrogen atom or a methyl radical, $R_2$ represents a methyl or ethyl radical, the nuclei A, B, C, D optionally carry one or more double bonds and are optionally substituted by one or more optionally protected hydroxyl or ketone functions, by one or more halogen atoms, by one or more alkyl or alkoxy radicals containing 1 to 4 carbon atoms, R represents a halogen atom, a hydroxyl radical, an alkoxy radical having 1 to 6 carbon atoms, an aralkoxy radical having 7 to 15 carbon atoms, a radical:

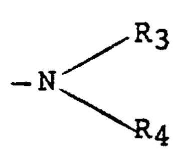

in which $R_3$ and $R_4$, identical or different, represent a hydrogen atom, an alkyl radical having 1 to 6 carbon atoms, an aralkyl radical having 7 to 15 carbon atoms or $R_3$ and $R_4$ form together with the nitrogen atom to which they are linked a heterocycle optionally containing another heteroatom chosen from nitrogen and oxygen atoms, or R represents an alkylthio radical having 1 to 6 carbon atoms, an

arylthio or aralkylthio radical having at most 15 carbon atoms, with the exception of the product in which $R_1$ and $R_2$ each represents a methyl radical, R represents a methoxy radical, the A nucleus has a 3-beta acetoxy position and the B nucleus carries a double bond 5(6), characterized in that a product of formula (II):

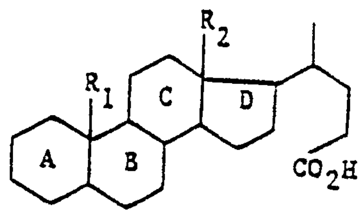

(II)

in which A, B, C, D, $R_1$ and $R_2$ have the meaning indicated in claim 1, is treated firstly with an acid halide formation agent, then with a tertiary base, then with thionyl chloride and finally optionally with water, an alkanol, an aralkanol, a primary or secondary amine of formula:

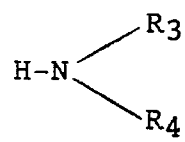

in which $R_3$ and $R_4$ have the meaning indicated in claim 1, an alkylthiol, an arylthiol or a aralkylthiol, in order to obtain a product of formula (III):

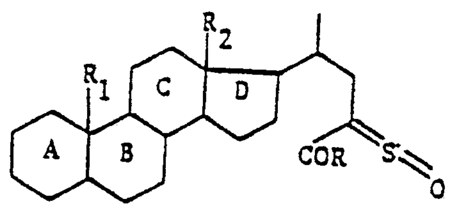

(III)

in which A, B, C, D, R, $R_1$ and $R_2$ have the meaning indicated in claim 1, which product of formula (III) is treated:

- either by an aqueous acid or an oxidizing agent,
- or by a halogenation reagent, then by a basic hydrolysis agent and a product of formula (I') obtained in which R represents a hydroxyl radical is optionally treated either by an alkanol, an aralkanol, a primary or secondary amine of formula:

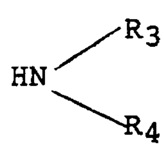

in which $R_3$ and $R_4$ have the meaning indicated in claim 1, an alkylthiol, an arylthiol or an aralkylthiol, or a product of formula (I') obtained in which R represents a hydroxyl radical is treated with an acid halide formation agent.

**6.** Use of the products of formula (I) for the preparation of products of formula (VI):

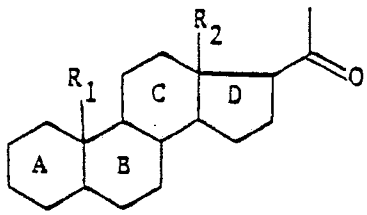

(VI)

in which A, B, C, D, $R_1$ and $R_2$ have the meaning indicated in claim 1, characterized in that a product of formula (I) is treated with a strong oxidizing agent in order to obtain a product of formula (IV):

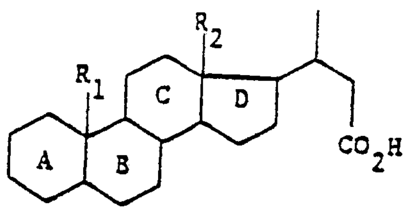

(IV)

in which A, B, C, D, $R_1$ and $R_2$ have the meaning indicated in claim 5, which product of formula (IV) is treated firstly with an acid halide formation agent, then with a tertiary base, then with thionyl chloride and with a halogenation agent and finally optionally with water, an alkanol, an aralkanol, a primary or secondary amine of formula:

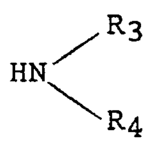

in which $R_3$ and $R_4$ have the meaning indicated in claim 1, an alkylthiol, an arylthiol or an aralkylthiol in order to obtain a product of formula (V):

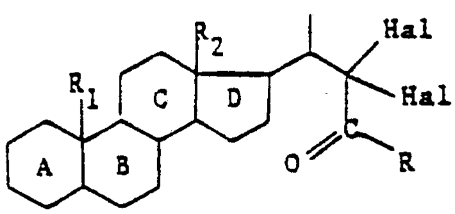

(V)

in which A, B, C, D, R, $R_1$, $R_2$ and R have the meaning indicated in claim 1 and Hal represents a halogen atom, which product of formula (V) is treated firstly with a dehydrogenation agent, then with an oxidizing cleaving agent, in order to obtain an expected product of formula (VI).

7. Process according to any one of claims 5 or 6, characterized in that the acid halide formation agent used is thionyl chloride.

8. As new industrial products, the products of formula (V) as defined in claim 6.

Claims for the following Contracting State : ES

1. Preparation process for the products of general formula (I):

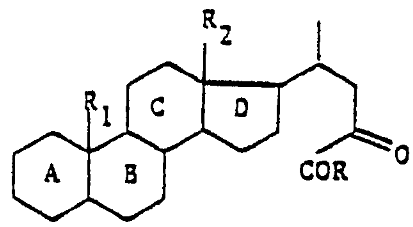

(I)

in which $R_1$ represents a hydrogen atom or a methyl radical, $R_2$ represents a methyl or ethyl radical, the nuclei A, B, C, D optionally carry one or more double bonds and are optionally substituted by one or more optionally protected hydroxyl or ketone functions, by one or more halogen atoms, by one or more alkyl or alkoxy radicals containing 1 to 4 carbon atoms, R represents a halogen atom, a hydroxyl radical, an alkoxy radical having 1 to 6 carbon atoms, an aralkoxy radical having 7 to 15 carbon atoms, a radical:

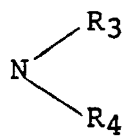

in which $R_3$ and $R_4$, identical or different, represent a hydrogen atom, an alkyl radical having 1 to 6 carbon atoms, an aralkyl radical having 7 to 15 carbon atoms or $R_3$ and $R_4$ form together with the nitrogen atom to which they are linked a heterocycle optionally containing another heteroatom chosen from nitrogen and oxygen atoms, or R represents an alkylthio radical having 1 to 6 carbon atoms, an arylthio or aralkylthio radical having at most 15 carbon atoms, with the exception of the product in which $R_1$ and $R_2$ each represents a methyl radical, R represents a methoxy radical, the A nucleus has a 3-beta acetoxy position and the B nucleus carries a double bond 5(6), characterized in that a product of formula (II):

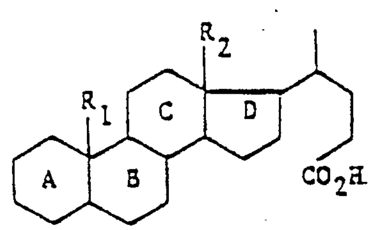

(II)

in which A, B, C, D, $R_1$ and $R_2$ have the meaning indicated above, is treated firstly with an acid halide formation agent, then with a tertiary base, then with thionyl chloride and finally optionally with water, an alkanol, an aralkanol, a primary or secondary amine of formula:

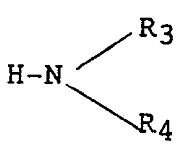

in which $R_3$ and $R_4$ have the meaning indicated above, an alkylthiol, an arylthiol or a aralkylthiol, in order to obtain a product of formula (III):

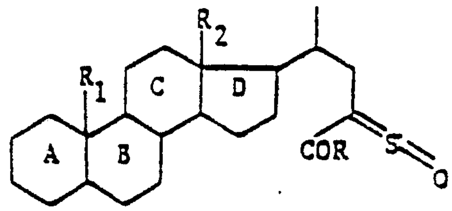

(III)

in which A, B, C, D, R, $R_1$ and $R_2$ have the meaning indicated above, which product of formula (III) is treated:

- either by an aqueous acid or an oxidizing agent,
- or by a halogenation reagent, then by a basic hydrolysis agent and a product of formula (I) obtained in which R represents a hydroxyl radical is optionally treated either by an alkanol, an aralkanol, a primary or secondary amine of formula:

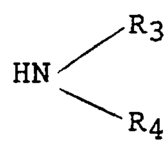

in which $R_3$ and $R_4$ have the meaning indicated above, an alkylthiol, an arylthiol or an aralkylthiol, or a product of formula (I) obtained in which R represents a hydroxyl radical is treated with an acid halide formation agent.

2. Process according to claim 1, characterized in that the product of formula (II) used at the start and the reagents are chosen in such a way that there are prepared products of formula (I) in which $R_1$ and $R_2$ each represents a methyl radical and the nuclei A, B, C, D carry in position 3 an optionally protected hydroxyl function and, optionally, one or more other functions chosen from the optionally protected hydroxyl functions in position 6, 7, 11 and 12 and the optionally protected ketone functions in position 7, 11 and 12 and R represents a hydroxy radical, an alkoxy radical having at most 4 carbon atoms or a radical:

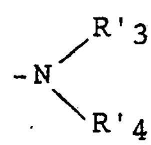

in which $R'_3$ and $R'_4$ represent a hydrogen atom, an alkyl radical having at most 4 carbon atoms or $R'_3$ and $R'_4$ form together with the nitrogen atom to which they are linked a piperidino, pyrrolidino or morpholino radical.

3. Process according to claim 1 or 2, characterized in that the product of formula (II) used at the start and the reagents are chosen in such a way that there are prepared products of formula (I) in which the nuclei A, B, cf D carry in position 3 an optionally protected hydroxyl radical and optionally one or more other functions chosen from the optionally protected hydroxyl functions in position 12 and the optionally protected ketone functions in position 11 or 12 and R represents a hydroxyl, methoxy or ethoxy radical or a morpholino radical.

4. Use of the products of formula (I) for the preparation of products of formula (VI):

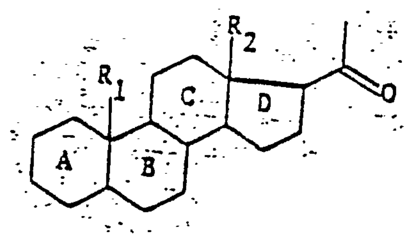

(VI)

in which A, B, C, D, $R_1$ and $R_2$ have the meaning indicated in claim 1, characterized in that a product of formula (I) is treated with a strong oxidizing agent in order to obtain a product of formula (IV):

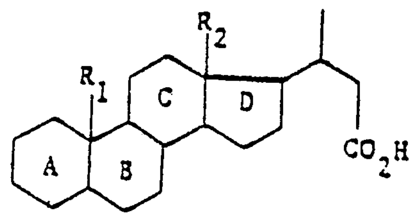

(IV)

in which A, B, C, D, $R_1$ and $R_2$ have the meaning indicated in claim 1, which product of formula (IV) is treated firstly with an acid halide formation agent, then with a tertiary base, then with thionyl chloride and with a halogenation agent and finally optionally with water, an alkanol, an aralkanol, a primary or secondary amine of formula:

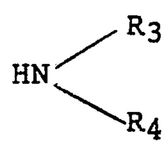

in which $R_3$ and $R_4$ have the meaning indicated in claim 1, an alkylthiol, an arylthiol or an aralkylthiol in order to obtain a product of formula (V):

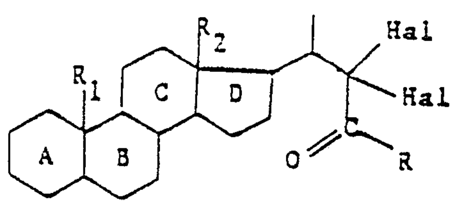

(V)

in which A, B, C, D, R, $R_1$, $R_2$ and R have the meaning indicated in claim 1 and Hal represents a halogen atom, which product of formula (V) is treated firstly with a dehydrogenation agent, then with an oxidizing cleaving agent in order to obtain an expected product of formula (VI).

**5.** Process according to any one of claims 1 or 4, characterized in that the acid halide formation agent used is thionyl chloride.

Claims for the following Contracting State : GR

**1.** Preparation process for the products of general formula (I):

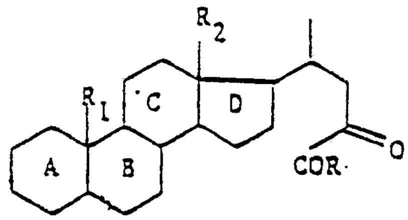

(I)

in which $R_1$ represents a hydrogen atom or a methyl radical, $R_2$ represents a methyl or ethyl radical, the nuclei A, B, C, D optionally carry one or more double bonds and are optionally substituted by one or more optionally protected hydroxyl or ketone functions, by one or more halogen atoms, by one or more alkyl or alkoxy radicals containing 1 to 4 carbon atoms, R represents a halogen atom, a hydroxyl radical, an alkoxy radical having 1 to 6 carbon atoms, an aralkoxy radical having 7 to 15 carbon atoms, a radical:

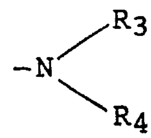

in which $R_3$ and $R_4$, identical or different, represent a hydrogen atom, an alkyl radical having 1 to 6 carbon atoms, an aralkyl radical having 7 to 15 carbon atoms or $R_3$ and $R_4$ form together with the nitrogen atom to which they are linked a heterocycle optionally containing another heteroatom chosen from nitrogen and oxygen atoms, or R represents an alkylthio radical having 1 to 6 carbon atoms, an arylthio or aralkylthio radical having at most 15 carbon atoms, with the exception of the product in which $R_1$ and $R_2$ each represents a methyl radical, R represents a methoxy radical, the A nucleus carries a 3-beta acetoxy position and the B nucleus carries a double bond 5(6), characterized in that a product of formula (II):

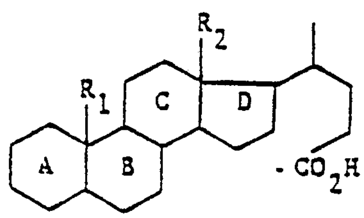

(II)

in which A, B, C, D, $R_1$ and $R_2$ have the meaning indicated above, is treated firstly with an acid halide formation agent, then with a tertiary base, then with thionyl chloride and finally optionally with water, an alkanol, an aralkanol, a primary or secondary amine of formula:

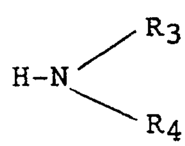

in which $R_3$ and $R_4$ have the meaning indicated above, an alkylthiol, an arylthiol or a aralkylthiol, in order to obtain a product of formula (III):

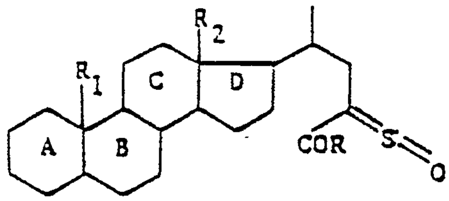

(III)

in which A, B, C, D, R, $R_1$ and $R_2$ have the meaning indicated above, which product of formula (III) is treated:

- either by an aqueous acid or an oxidizing agent,
- or by a halogenation reagent, then by a basic hydrolysis agent and a product of formula (I) obtained in which R represents a hydroxyl radical is optionally treated either by an alkanol, an aralkanol, a primary or secondary amine of formula:

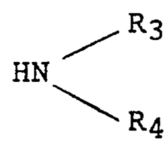

in which $R_3$ and $R_4$ have the meaning indicated above, an alkylthiol, an arylthiol or an aralkylthiol, or a product of formula (I) obtained in which R represents a hydroxyl radical is treated with an acid halide formation agent.

**2.** Process according to claim 1, characterized in that the product of formula (II) used at the start and the reagents are chosen in such a way that there are prepared products of formula (I) in which $R_1$ and $R_2$ each represents a methyl radical and the nuclei A, B, C, D carry in position 3 an optionally protected hydroxyl function and, optionally, one or more other functions chosen from the optionally protected hydroxyl functions in position 6, 7, 11 and 12 and the optionally protected ketone functions in position 7, 11 and 12 and R represents a hydroxy radical, an alkoxy radical having at most 4 carbon atoms or a radical:

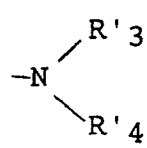

in which $R'_3$ and $R'_4$ represent a hydrogen atom, an alkyl radical having at most 4 carbon atoms or $R'_3$ and $R'_4$ form together with the nitrogen atom to which they are linked a piperidino, pyrrolidino or morpholino radical.

**3.** Process according to claim 1 or 2, characterized in that the product of formula (II) used at the start and the reagents are chosen in such a way that there are prepared products of formula (I) in which the nuclei A, B, C, D carry in position 3 an optionally protected hydroxyl radical and optionally one or more other functions chosen from the optionally protected hydroxyl functions in position 12 and the optionally protected ketone functions in position 11 or 12 and R represents a hydroxyl, methoxy or ethoxy radical or a morpholino radical.

**4.** Process according to any one of claims 1 to 3, characterized in that the products of general formula (I) are prepared of which the names follow:

- (3-alpha, 5-beta)-4-3-(acetyloxy)-11,23,24-trioxo-cholan-24-yl morpholine.
- (3-alpha, 5-beta)-11,23-dioxo-3-hydroxy-cholan-24-oic acid.
- Methyl ester of 3-alpha-hydroxy-11,23-dioxo-5-beta-cholan-24-oic acid.
- (3-alpha, 5-beta)-3-(acetyloxy)-11,23-dioxo-cholan-24-oic acid.

5. Use of the products of formula (I) for the preparation of products of formula (VI):

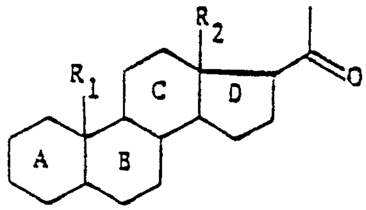

(VI)

in which A, B, C, D, $R_1$ and $R_2$ have the meaning indicated in claim 1, characterized in that a product of formula (I) is treated with a strong oxidizing agent in order to obtain a product of formula (IV):

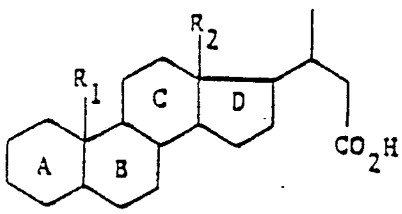

(IV)

in which A, B, C, D, $R_1$ and $R_2$ have the meaning indicated in claim 1, which product of formula (IV) is treated firstly with an acid halide formation agent, then with a tertiary base, then with thionyl chloride and with a halogenation agent and finally optionally with water, an alkanol, an aralkanol, a primary or secondary amine of formula:

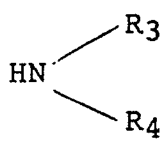

in which $R_3$ and $R_4$ have the meaning indicated in claim 1, an alkylthiol, an arylthiol or an aralkylthiol in order to obtain a product of formula (V):

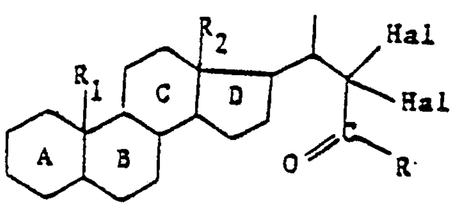

(V)

in which A, B, C, D, R, $R_1$, $R_2$ and R have the meaning indicated in claim 1 and Hal represents a halogen atom, which product of formula (V) is treated firstly with a dehydrogenation agent, then with an oxidizing cleaving agent in order to obtain an expected product of formula (VI).

6. Process according to any one of claims 1 or 5, characterized in that the acid halide formation agent used is thionyl chloride.

7. As new industrial products, the products of formula (V) as defined in claim 5.

**Ansprüche**

1. Verbindungen der allgemeinen Formel I:

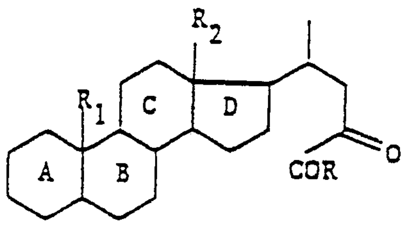

(I)

worin $R_1$ ein Wasserstoffatom oder eine Methylgruppe bedeutet, $R_2$ eine Methyl- oder Ethylgruppe bedeutet, die Ringe A, B, C, D gegebenenfalls eine oder mehrere Doppelbindungen aufweisen und gegebenenfalls substituiert sind durch eine oder mehrere gegebenenfalls geschützte Hydroxy- oder Ketofunktionen, durch ein oder mehrere Halogenatome, durch eine oder mehrere Alkyl- oder Alkoxygruppen mit 1 bis 4 Kohlenstoffatomen, R ein Halogenatom, eine Hydroxygruppe, eine Alkoxygruppe mit 1 bis 6 Kohlenstoffatomen, eine Aralkoxygruppe mit 7 bis 15 Kohlenstoffatomen, eine Gruppe:

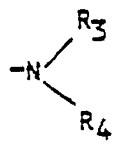

bedeutet, worin $R_3$ und $R_4$, die gleich oder verschieden sind, ein Wasserstoffatom, eine Alkylgruppe mit 1 bis 6 Kohlenstoffatomen, eine Aralkylgruppe mit 7 bis 15 Kohlenstoffatomen bedeuten, oder $R_3$ und $R_4$ zusammen mit dem Stickstoffatom, an das sie gebunden sind, einen Heterocyclus bilden, der gegebenenfalls ein weiteres Heteroatom, ausgewählt aus Stickstoff und Sauerstoff, aufweist, oder R eine Alkylthiogruppe mit 1 bis 6 Kohlenstoffatomen, eine Arylthio- oder Aralkylthiogruppe mit höchstens 15 Kohlenstoffatomen bedeutet, ausgenommen eine Verbindung, worin $R_1$ und $R_2$ jeweils eine Methylgruppe bedeuten, R eine Methoxygruppe bedeutet, der Ring A eine 3-$\beta$-Acetoxygruppe aufweist, und der Ring B eine 5(6)-Doppelbindung aufweist, und wobei der Ring C entweder in 11-Position eine gegebenenfalls geschützte Ketofunktion oder in 12-Position eine gegebenenfalls geschützte Hydroxyfunktion aufweist.

2. Verbindungen der allgemeinen Formel I, wie in Anspruch 1 definiert, worin $R_1$ und $R_2$ jeweils eine Methylgruppe bedeuten, und die Ringe A, B, C, D eine gegebenenfalls geschützte Hydroxyfunktion in 3-Position und gegebenenfalls eine oder mehrere weitere Funktionen, ausgewählt aus gegebenenfalls geschützten Hydroxyfunktionen in 6-, 7- und 12-Position und gegebenenfalls geschützten Ketofunktionen in 7- und 11-Position, aufweisen, und R eine Hydroxygruppe, eine Alkoxygruppe mit höchstens 4 Kohlenstoffatomen oder eine Gruppe:

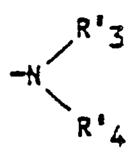

bedeutet, worin $R'_3$ und $R'_4$ ein Wasserstoffatom, eine Alkylgruppe mit höchstens 4 Kohlenstoffatomen bedeuten, oder $R'_3$ und $R'_4$ zusammen mit dem Stickstoffatom, an das sie gebunden sind, eine Piperidino-, Pyrrolidino- oder Morpholinogruppe bedeuten.

3. Verbindungen der allgemeinen Formel I, wie in einem der Ansprüche 1 oder 2 definiert, die in 3-Position eine gegebenenfalls geschützte Hydroxygruppe aufweisen, und R eine Hydroxy-, Methoxy- oder Ethoxygruppe oder eine Morpholinogruppe bedeutet.

**4.** Verbindungen der allgemeinen Formel I, wie in einem der Ansprüche 1 bis 3 definiert, entsprechend den folgenden Formeln:

- ($3\alpha$, $5\beta$)-4-[3-(Acetyloxy)-11,23,24-trioxo-cholan-24-yl]-morpholin;
- ($3\alpha$, $5\beta$)-11,23-Dioxo-3-hydroxy-cholan-24-säure;
- Methylester der $3\alpha$-Hydroxy-11,23-dioxo-$5\beta$-cholan-24-säure;
- ($3\alpha$, $5\beta$)-3-(Acetyloxy)-11,23-dioxo-cholan-24-säure.

**5.** Verfahren zur Herstellung von Verbindungen der Formel I':

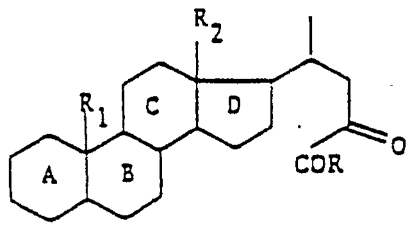

(I')

worin $R_1$ ein Wasserstoffatom oder eine Methylgruppe bedeutet, $R_2$ eine Methyl- oder Ethylgruppe bedeutet, die Ringe A, B, C, D gegebenenfalls eine oder mehrere Doppelbindungen aufweisen und gegebenenfalls substituiert sind durch eine oder mehrere gegebenenfalls geschützte Hydroxy- oder Ketofunktionen, durch eine oder mehrere Halogenatome, durch eine oder mehrere Alkyl- oder Alkoxygruppen mit 1 bis 4 Kohlenstoffatomen, R ein Halogenatom, eine Hydroxygruppe, eine Alkoxygruppe mit 1 bis 6 Kohlenstoffatomen, eine Aralkoxygruppe mit 7 bis 15 Kohlenstoffatomen, eine Gruppe:

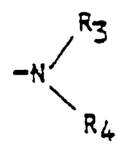

bedeutet, worin $R_3$ und $R_4$, die gleich oder verschieden sind, ein Wasserstoffatom, eine Alkylgruppe mit 1 bis 6 Kohlenstoffatomen, eine Aralkylgruppe mit 7 bis 15 Kohlenstoffatomen bedeuten, oder $R_3$ und $R_4$ zusammen mit dem Stickstoffatom, an das sie gebunden sind, einen Heterocyclus bilden, der gegebenenfalls ein weiteres Heteroatom, ausgewählt aus Stickstoff und Sauerstoff, aufweist, oder R eine Alkylthiogruppe mit 1 bis 6 Kohlenstoffatomen, eine Arylthio- oder Aralkylthiogruppe mit höchstens 15 Kohlenstoffatomen bedeutet, ausgenommen die Verbindung, worin $R_1$ und $R_2$ jeweils eine Methylgruppe bedeuten, R eine Methoxygruppe bedeutet, der Ring A eine 3-$\beta$-Acetoxygruppe aufweist, und der Ring B eine 5(6)-Doppelbindung aufweist, dadurch gekennzeichnet, daß man eine Verbindung der Formel II:

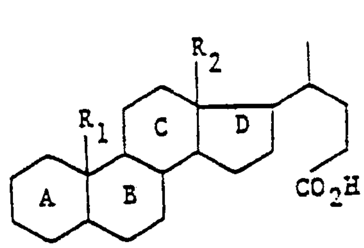

(II)

worin A, B, C, D, $R_1$ und $R_2$ die in Anspruch 1 angegebene Bedeutung besitzen, zunächst mit einem ein Säurehalogenid bildenden Reagens, sodann mit einer tertiären Base, dann mit Thionylchlorid und schließlich gegebenenfalls mit Wasser, einem Alkanol, einem Aralkanol, einem primären oder sekundären Amin der Formel:

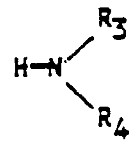

worin $R_3$ und $R_4$ die in Anspruch 1 angegebene Bedeutung besitzen, einem Alkylthiol, einem Arylthiol oder einem Aralkylthiol behandelt, um eine Verbindung der Formel III:

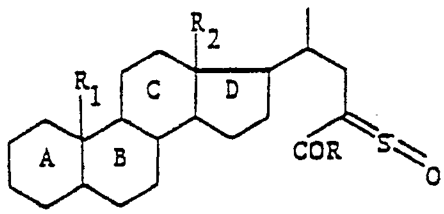

(III)

zu erhalten, worin A, B, C, D, R, $R_1$ und $R_2$ die in Anspruch 1 angegebene Bedeutung besitzen, diese Verbindung der Formel III entweder behandelt:

- mit einer wäßrigen Säure oder einem Oxidationsmittel,
- oder mit einem Halogenierungsmittel, dann mit einem basischen Hydrolysereagens, und man gegebenenfalls eine erhaltene Verbindung der Formel I', worin R eine Hydroxygruppe bedeutet, mit einem Alkanol, einem Aralkanol, einem primären oder sekundären Amin der Formel:

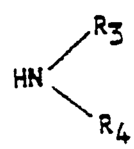

worin $R_3$ und $R_4$ die in Anspruch 1 angegebene Bedeutung besitzen, einem Alkylthiol, einem Arylthiol oder einem Aralkylthiol behandelt, oder man eine erhaltene Verbindung der Formel I', worin R eine Hydroxygruppe bedeutet, mit einem ein Säurehalogenid bildenden Reagens behandelt.

6. Verwendung der Verbindungen der Formel I zur Herstellung von Verbindungen der Formel VI:

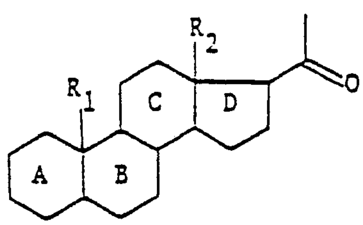

(VI)

worin A, B, C, D, $R_1$ und $R_2$ die in Anspruch 1 angegebene Bedeutung besitzen, dadurch gekennzeichnet, daß man eine Verbindung der Formel I mit einem starken Oxidationsmittel behandelt, um eine Verbindung der Formel IV zu erhalten:

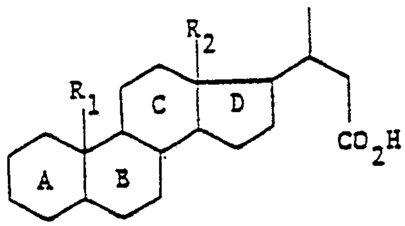

(IV)

worin A, B, C, D, $R_1$ und $R_2$ die in Anspruch 5 angegebene Bedeutung besitzen, diese Verbindung der Formel IV zunächst mit einem ein Säurehalogenidbildenden Reagens, sodann mit einer tertiären Base, dann mit Thionylchlorid und mit einem Halogenierungsmittel und schließlich gegebenenfalls mit Wasser, einem Alkanol, einem Aralkanol, einem primären oder sekundären Amin der Formel:

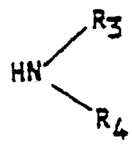

worin $R_3$ und $R_4$ die in Anspruch 1 angegebene Bedeutung besitzen, einem Alkylthiol, einem Arylthiol oder einem Aralkylthiol behandelt, um eine Verbindung der Formel V:

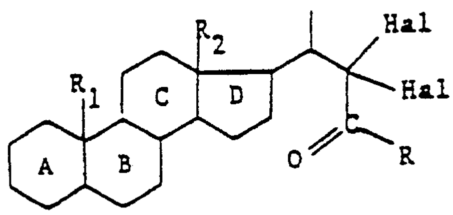

(V)

zu erhalten, worin A, B, C, D, R, $R_1$ und $R_2$ die in Anspruch 1 angegebene Bedeutung besitzen, und Hal ein Halogenatom bedeutet, diese Verbindung der Formel V zunächst mit einem Halogenwasserstoff abspaltenden Mittel, dann mit einem Reagens zur oxidativen Spaltung behandelt, um eine gewünschte Verbindung der Formel VI zu erhalten.

7. Verfahren gemäß einem der Ansprüche 5 oder 6, dadurch gekennzeichnet, daß das verwendete, ein Säurehalogenid bildende Reagens Thionylchlorid ist.

8. Verbindungen der Formel V, wie in Anspruch 6 definiert, als neue industrielle Verbindungen.

Patentansprüche für folgenden Vertragsstaat : ES

1. Verfahren zur Herstellung von Verbindungen der allgemeinen Formel I

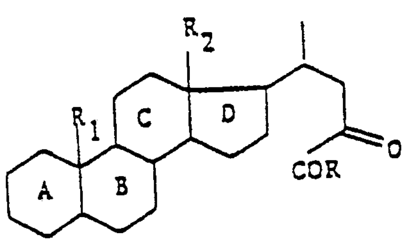

(I)

worin $R_1$ ein Wasserstoffatom oder eine Methylgruppe bedeutet, $R_2$ eine Methyl- oder Ethylgruppe

bedeutet, die Ringe A, B, C, D gegebenenfalls eine oder mehrere Doppelbindungen aufweisen und gegebenenfalls substituiert sind durch eine oder mehrere gegebenenfalls geschützte Hydroxy- oder Ketofunktionen, durch ein oder mehrere Halogenatome, durch eine oder mehrere Alkyl- oder Alkoxygruppen mit 1 bis 4 Kohlenstoffatomen, R ein Halogenatom, eine Hydroxygruppe, eine Alkoxygruppe mit 1 bis 6 Kohlenstoffatomen, eine Aralkoxygruppe mit 7 bis 15 Kohlenstoffatomen, eine Gruppe:

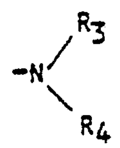

bedeutet, worin $R_3$ und $R_4$, die gleich oder verschieden sind, ein Wasserstoffatom, eine Alkylgruppe mit 1 bis 6 Kohlenstoffatomen, eine Aralkylgruppe mit 7 bis 15 Kohlenstoffatomen bedeuten, oder $R_3$ und $R_4$ zusammen mit dem Stickstoffatom, an das sie gebunden sind, einen Heterocyclus bilden, der gegebenenfalls ein weiteres Heteroatom, ausgewählt aus Stickstoff und Sauerstoff, aufweist, oder R eine Alkylthiogruppe mit 1 bis 6 Kohlenstoffatomen, eine Arylthio- oder Aralkylthiogruppe mit höchstens 15 Kohlenstoffatomen bedeutet, ausgenommen eine Verbindung, worin $R_1$ und $R_2$ jeweils eine Methylgruppe bedeuten, R eine Methoxygruppe bedeutet, der Ring A eine 3-$\beta$-Acetoxygruppe aufweist, und der Ring B eine 5(6)-Doppelbindung aufweist, dadurch gekennzeichnet, daß man eine Verbindung der Formel II:

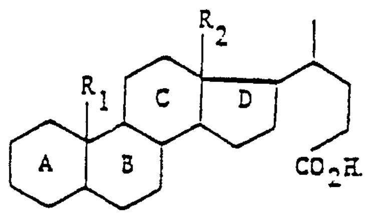

(II)

worin A, B, C, D, $R_1$ und $R_2$ die vorstehende Bedeutung besitzen, zunächst mit einem ein Säurehalogenid bildenden Reagens, sodann mit einer tertiären Base, dann mit Thionylchlorid und schließlich gegebenenfalls mit Wasser, einem Alkanol, einem Aralkanol, einem primären oder sekundären Amin der Formel:

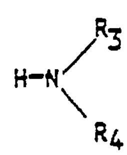

worin $R_3$ und $R_4$ die vorstehend angegebene Bedeutung besitzen, einem Alkylthiol, einem Arylthiol oder einem Aralkylthiol behandelt, um eine Verbindung der Formel III:

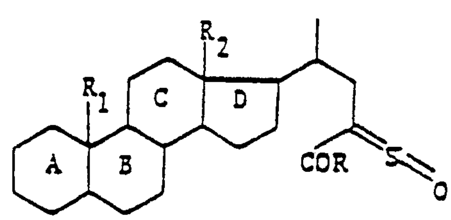

(III)

zu erhalten, worin A, B, C, D, R, $R_1$ und $R_2$ die vorstehend angegebene Bedeutung besitzen, diese Verbindung der Formel III entweder behandelt:

- mit einer wäßrigen Säure oder einem Oxidationsmittel,
- oder mit einem Halogenierungsmittel, dann mit einem basischen Reagens zur Hydrolyse und gegebenenfalls eine erhaltene Verbindung der Formel I, worin R eine Hydroxygruppe bedeutet, mit einem Alkanol, einem Aralkanol, einem primären oder sekundären Amin der Formel:

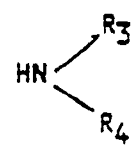

worin $R_3$ und $R_4$ die vorstehend angegebene Bedeutung besitzen, einem Alkylthiol, einem Arylthiol oder einem Aralkylthiol behandelt, oder eine erhaltene Verbindung der Formel I, worin R eine Hydroxygruppe bedeutet, mit einem ein Säurehalogenid bildenden Reagens behandelt.

**2.** Verfahren nach Anspruch 1, dadurch gekennzeichnet, daß das verwendete Ausgangsprodukt der Formel II und die Reaktionspartner derart gewählt sind, daß man Verbindungen der Formel I herstellt, worin $R_1$ und $R_2$ jeweils eine Methylgruppe bedeuten, und die Ringe A, B, C, D in 3-Position eine gegebenenfalls geschützte Hydroxyfunktion und gegebenenfalls eine oder mehrere weitere Funktionen, ausgewählt aus gegebenenfalls geschützten Hydroxyfunktionen in 6-, 7-, 11- und 12-Position und gegebenenfalls geschützten Ketofunktionen in 7-, 11- und 12-Position, aufweisen, und R eine Hydroxygruppe, eine Alkoxygruppe mit höchstens 4 Kohlenstoffatomen oder eine Gruppe:

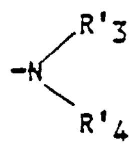

bedeuten, worin $R'_3$ und $R'_4$ ein Wasserstoffatom, eine Alkylgruppe mit höchstens 4 Kohlenstoffatomen bedeuten, oder $R'_3$ und $R'_4$ zusammen mit dem Stickstoffatom, an das sie gebunden sind, eine Piperidino-, Pyrrolidino- oder Morpholinogruppe bilden.

**3.** Verfahren nach den Ansprüchen 1 oder 2, dadurch gekennzeichnet, daß das verwendete Ausgangsprodukt der Formel II und die Reaktionspartner derart gewählt sind, daß man Verbindungen der Formel I herstellt, worin die Ringe A, B, C, D in 3-Position eine gegebenenfalls geschützte Hydroxylgruppe und gegebenenfalls eine oder mehrere weitere Funktionen, ausgewählt aus gegebenenfalls geschützten Hydroxyfunktionen in 12-Position und gegebenenfalls geschützten Ketofunktionen in 11- oder 12-Position aufweisen, und R eine Hydroxyl-, Methoxy- oder Ethoxygruppe oder eine Morpholinogruppe bedeuten.

**4.** Verwendung der Verbindungen der Formel I zur Herstellung von Verbindungen der Formel VI:

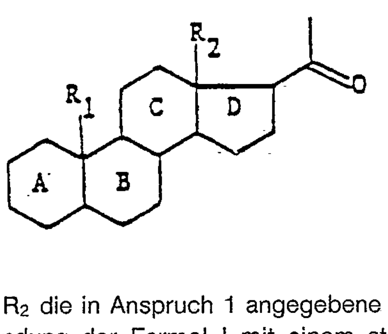

(VI)

worin A, B, C, D, $R_1$ und $R_2$ die in Anspruch 1 angegebene Bedeutung besitzen, dadurch gekennzeichnet, daß man eine Verbindung der Formel I mit einem starken Oxidationsmittel behandelt, um eine

Verbindung der Formel IV:

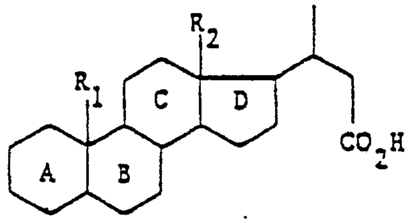

(IV)

zu erhalten, worin A, B, C, D, $R_1$ und $R_2$ die in Anspruch 1 angegebene Bedeutung besitzen, man diese Verbindung der Formel IV zunächst mit einem ein Säurehalogenid bildenden Reagens, sodann mit einer tertiären Base, dann mit Thionylchlorid und mit einem Halogenierungsmittel und schließlich gegebenenfalls mit Wasser, einem Alkanol, einem Aralkanol, einem primären oder sekundären Amin der Formel:

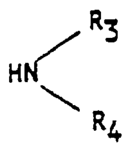

worin $R_3$ und $R_4$ die in Anspruch 1 angegebene Bedeutung besitzen, einem Alkylthiol, einem Arylthiol oder einem Aralkylthiol behandelt, um eine Verbindung der Formel V:

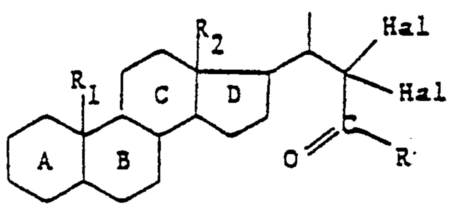

(V)

zu erhalten, worin A, B, C, D, R, $R_1$ und $R_2$ die in Anspruch 1 angegebene Bedeutung besitzen, und Hal ein Halogenatom bedeutet, diese Verbindung der Formel V zunächst mit einem Halogenwasserstoff abspaltenden Mittel, dann mit einem Reagens zur oxidativen Spaltung behandelt, um eine gewünschte Verbindung der Formel VI zu erhalten.

**5.** Verfahren gemäß einem der Ansprüche 1 bis 4, dadurch gekennzeichnet, daß das verwendete, ein Säurehalogenid bildende Reagens Thionylchlorid ist.

Patentansprüche für folgenden Vertragsstaat : GR

**1.** Verfahren zur Herstellung von Verbindungen der allgemeinen Formel I

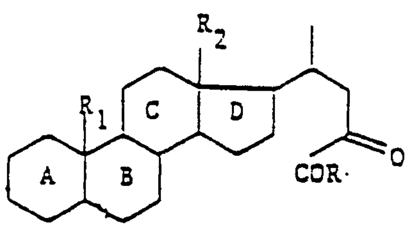

(I)

worin $R_1$ ein Wasserstoffatom oder eine Methylgruppe bedeutet, $R_2$ eine Methyl- oder Ethylgruppe

bedeutet, die Ringe A, B, C, D gegebenenfalls eine oder mehrere Doppelbindungen aufweisen und gegebenenfalls substituiert sind durch eine oder mehrere gegebenenfalls geschützte Hydroxy- oder Ketofunktionen, durch ein oder mehrere Halogenatome, durch eine oder mehrere Alkyl- oder Alkoxygruppen mit 1 bis 4 Kohlenstoffatomen, R ein Halogenatom, eine Hydroxygruppe, eine Alkoxygruppe mit 1 bis 6 Kohlenstoffatomen, eine Aralkoxygruppe mit 7 bis 15 Kohlenstoffatomen, eine Gruppe:

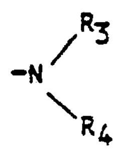

bedeutet, worin $R_3$ und $R_4$, die gleich oder verschieden sind, ein Wasserstoffatom, eine Alkylgruppe mit 1 bis 6 Kohlenstoffatomen, eine Aralkylgruppe mit 7 bis 15 Kohlenstoffatomen bedeuten, oder $R_3$ und $R_4$ zusammen mit dem Stickstoffatom, an das sie gebunden sind, einen Heterocyclus bilden, der gegebenenfalls ein weiteres Heteroatom, ausgewählt aus Stickstoff und Sauerstoff, aufweist, oder R eine Alkylthiogruppe mit 1 bis 6 Kohlenstoffatomen, eine Arylthio- oder Aralkylthiogruppe mit höchstens 15 Kohlenstoffatomen bedeutet, ausgenommen eine Verbindung, worin $R_1$ und $R_2$ jeweils eine Methylgruppe bedeuten, R eine Methoxygruppe bedeutet, der Ring A eine 3-$\beta$-Acetoxygruppe aufweist, und der Ring B eine 5(6)-Doppelbindung aufweist, dadurch gekennzeichnet, daß man eine Verbindung der Formel II:

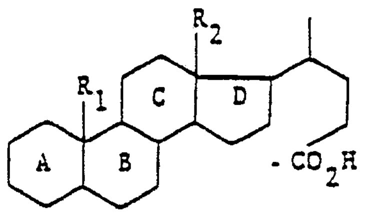

(II)

worin A, B, C, D, $R_1$ und $R_2$ die vorstehende Bedeutung besitzen, zunächst mit einem ein Säurehalogenid bildenden Reagens, sodann mit einer tertiären Base, dann mit Thionylchlorid und schließlich gegebenenfalls mit Wasser, einem Alkanol, einem Aralkanol, einem primären oder sekundären Amin der Formel:

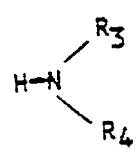

worin $R_3$ und $R_4$ die vorstehend angegebene Bedeutung besitzen, einem Alkylthiol, einem Arylthiol oder einem Aralkylthiol behandelt, um eine Verbindung der Formel III:

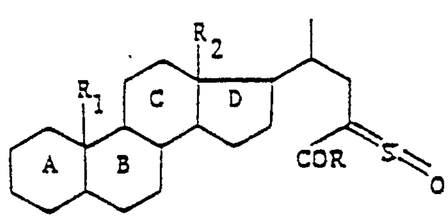

(III)

zu erhalten, worin A, B, C, D, R, $R_1$ und $R_2$ die vorstehend angegebene Bedeutung besitzen, diese Verbindung der Formel III entweder behandelt:

- mit einer wäßrigen Säure oder einem Oxidationsmittel,

- oder mit einem Halogenierungsmittel, dann mit einem basischen Reagens zur Hydrolyse und gegebenenfalls eine erhaltene Verbindung der Formel I, worin R eine Hydroxygruppe bedeutet, mit einem Alkanol, einem Aralkanol, einem primären oder sekundären Amin der Formel:

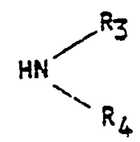

worin $R_3$ und $R_4$ die vorstehend angegebene Bedeutung besitzen, einem Alkylthiol, einem Arylthiol oder einem Aralkylthiol behandelt, oder eine erhaltene Verbindung der Formel I, worin R eine Hydroxygruppe bedeutet, mit einem ein Säurehalogenid bildenden Reagens behandelt.

2. Verfahren nach Anspruch 1, dadurch gekennzeichnet, daß das verwendete Ausgangsprodukt der Formel II und die Reaktionspartner derart gewählt sind, daß man Verbindungen der Formel I herstellt, worin $R_1$ und $R_2$ jeweils eine Methylgruppe bedeuten, und die Ringe A, B, C, D in 3-Position eine gegebenenfalls geschützte Hydroxyfunktion und gegebenenfalls eine oder mehrere weitere Funktionen, ausgewählt aus gegebenenfalls geschützten Hydroxyfunktionen in 6-, 7-, 11- und 12-Position und gegebenenfalls geschützten Ketofunktionen in 7-, 11- und 12-Position, aufweisen, und R eine Hydroxygruppe, eine Alkoxygruppe mit höchstens 4 Kohlenstoffatomen oder eine Gruppe:

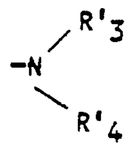

bedeutet, worin $R'_3$ und $R'_4$ ein Wasserstoffatom, eine Alkylgruppe mit höchstens 4 Kohlenstoffatomen bedeuten, oder $R'_3$ und $R'_4$ zusammen mit dem Stickstoffatom, an das sie gebunden sind, eine Piperidino-, Pyrrolidino- oder Morpholinogruppe bilden.

3. Verfahren nach den Ansprüchen 1 oder 2, dadurch gekennzeichnet, daß das verwendete Ausgangsprodukt der Formel II und die Reaktionspartner derart gewählt sind, daß man Verbindungen der Formel I herstellt, worin die Ringe A, B, C, D in 3-Position eine gegebenenfalls geschützte Hydroxylgruppe und gegebenenfalls eine oder mehrere weitere Funktionen, ausgewählt aus gegebenenfalls geschützten Hydroxyfunktionen in 12-Position und gegebenenfalls geschützten Ketofunktionen in 11- oder 12-Position aufweisen, und R eine Hydroxyl-, Methoxy- oder Ethoxygruppe oder eine Morpholinogruppe bedeutet.

4. Verfahren nach einem der Ansprüche 1 bis 3, dadurch gekennzeichnet, daß man die Verbindungen der allgemeinen Formel I mit folgenden Namen herstellt:
   - (3α, 5β)-4-[3-(Acetyloxy)-11,23,24-trioxo-cholan-24-yl]-morpholin;
   - (3α, 5β)-11,23-Dioxo-3-hydroxy-cholan-24-säure;
   - Methylester der 3α-Hydroxy-11,23-dioxo-5β-cholan-24-säure;
   - (3α, 5β)-3-(Acetyloxy)-11,23-dioxo-cholan-24-säure.

5. Verwendung der Verbindungen der Formel I zur Herstellung von Verbindungen der Formel VI:

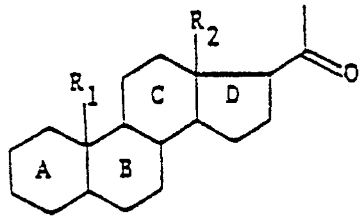

(VI)

worin A, B, C, D, $R_1$ und $R_2$ die in Anspruch 1 angegebene Bedeutung besitzen, dadurch gekennzeichnet, daß man eine Verbindung der Formel I mit einem starken Oxidationsmittel behandelt, um eine Verbindung der Formel IV:

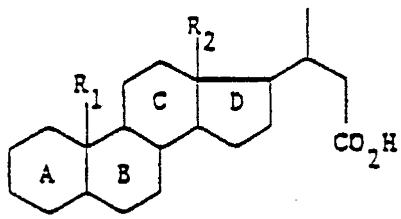

(IV)

zu erhalten, worin A, B, C, D, $R_1$ und $R_2$ die in Anspruch 1 angegebene Bedeutung besitzen, man diese Verbindung der Formel IV zunächst mit einem ein Säurehalogenid bildenden Reagens, sodann mit einer tertiären Base, dann mit Thionylchlorid und mit einem Halogenierungsmittel und schließlich gegebenenfalls mit Wasser, einem Alkanol, einem Aralkanol, einem primären oder sekundären Amin der Formel:

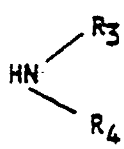

worin $R_3$ und $R_4$ die in Anspruch 1 angegebene Bedeutung besitzen, einem Alkylthiol, einem Arylthiol oder einem Aralkylthiol behandelt, um eine Verbindung der Formel V:

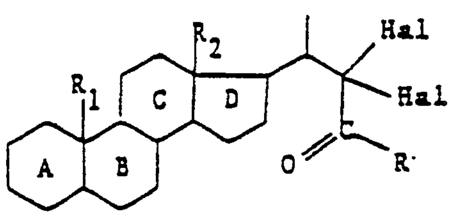

(V)

zu erhalten, worin A, B, C, D, R, $R_1$ und $R_2$ die in Anspruch 1 angegebene Bedeutung besitzen, und Hal ein Halogenatom bedeutet, diese Verbindung der Formel V zunächst mit einem Halogenwasserstoff abspaltenden Mittel, dann mit einem Reagens zur oxidativen Spaltung behandelt, um eine gewünschte Verbindung der Formel VI zu erhalten.

6. Verfahren nach einem der Ansprüche 1 oder 5, dadurch gekennzeichnet, daß das verwendete, ein Säurehalogenid bildende Reagens Thionylchlorid ist.

7. Verbindungen der Formel V, wie in Anspruch 5 definiert, als neue industrielle Verbindungen.